# EUROPEAN PATENT APPLICATION

(11) **EP 4 190 818 A1**
(43) Date of publication of application: **07.06.2023**
(21) Application number: 21850196.3
(22) Date of filing: 27.07.2021
(51) Int. Cl.: C07K 16/32, A61K 39/395, A61P 35/00

(54) **ANTI-HER2 ANTIBODY AND USE THEREOF**

(30) Priority: 28.07.2020 CN 202010739418
(71) Applicant: Bio-Thera Solutions, Ltd., Guangzhou, Guangdong 510530 (CN)
(72) Inventor: MEI, Xingxing, Guangzhou, Guangdong 510530 (CN); TANG, Weijia, Guangzhou, Guangdong 510530 (CN); YU, Jin-Chen, Guangzhou, Guangdong 510530 (CN); FENG, Cuiying, Guangzhou, Guangdong 510530 (CN); LIU, Yujie, Guangzhou, Guangdong 510530 (CN)
(74) Representative: karo IP
(86) International application number: PCT/CN2021/108724
(87) International publication number: WO 2022/022526

(57) **Abstract**

The present invention provides an anti-HER2 antibody and use thereof, wherein the antibody provided by the present invention can be used for the treatment of HER2-positive diseases, and can also be used for the diagnosis and prognosis of HER2-positive diseases. The Antibody BAT0303F disclosed herein is expressed by a host cell with fucosyltransferase knocked out, and can enhance ADCC effect of the antibody.

## Description

### TECHNICAL FIELD

The present invention belongs to the technical field of bio-pharmaceuticals, and particularly relates to an anti-HER2 antibody and use thereof.

### BACKGROUND

Human epidermal growth factor receptor 2 (HER-2, also known as ErbB-2) is a single-chain transmembrane glycoprotein with a molecular weight of 185 KD, wherein the extracellular part is a ligand-binding region including four subregions I to IV, and a spatial position of the extracellular segment in the resting state is shown as an activated state similar to that after ligand binding, making it a dimer molecule in the absence of a natural ligand having high affinity; and the intracellular part has tyrosine kinase activity. HER2 (i.e., HER-2) is a member of the HER/erbB family, which further comprises HER1 (i.e., ErbB1 or EGFR), HER3 (ErbB3) and HER4 (ErbB4). The HER2 encoding gene is located on chromosome 17 and encodes type I transmembrane growth factor receptor tyrosine kinase. In the process of activating an HER/erbB family receptor, after binding to a ligand, the extracellular segment of the receptor induces the formation of receptor dimers, activates intracellular tyrosine kinases, activates downstream signaling pathways dominated by Ras/Raf/MEK/Erk and P13K/Akt, and participates in biological functions such as cell proliferation and apoptosis regulation. HER2 overexpressed in tumors can form dimers or heterodimers with itself, or with other members of the family in the case of ligand deletion, leading to abnormal activation of signaling pathways within tumor cells.

In normal body tissues, the HER2 receptor is mainly expressed during embryonic development and is low-expressed in a few mature body tissues in adulthood. Studies have found that HER2 is overexpressed in 25%-30% of breast cancer cells, about 20% of gastric cancer cells, and about 10% of ovarian cancer cells.

Trastuzumab can selectively act on the extracellular IV region of HER-2 protein and inhibit the formation of tumor blood vessels, delaying the growth and progression of tumors. The current research suggests that the mechanism of action of trastuzumab is as follows: (1) binding to Fc-γ receptor, thereby activating antibody-dependent cell-mediated cytotoxicity (ADCC), generating body immunity and killing HER-2 positive tumor cells; (2) inhibiting the homodimerization of HER-2 receptor by acting on the extracellular domain of the HER-2 receptor, thereby blocking the transduction of downstream signals of the receptor and inhibiting the survival and proliferation of tumor cells; (3) reducing the density of tumor blood vessels by inhibiting the formation of tumor vascular epidermal growth factor; (4) inducing self-degradation and death of HER-2 positive cancer cells; (5) acting on PI3K-AKT signaling pathway, thereby blocking the transduction of downstream signals of tumor cells; and (6) reducing the expression of cyclin D1 by inhibiting P27kipl formation, promoting apoptosis and arresting the cell cycle. As a novel humanized monoclonal antibody, pertuzumab (Perjeta^{®}) is different from the action site (region IV) of trastuzumab, which binds to the extracellular domain II of an HER-2 receptor, blocks the heterodimerization of HER-2 and other EGFR receptor families, and inhibits the proliferation and survival of tumor cells related to HER-2 receptor activity.

### SUMMARY

The present invention provides an antibody that can specifically bind to HER2. The antibody provided by the present invention can be used for the treatment of HER2-positive diseases, and can also be used for the diagnosis and prognosis of HER2-positive diseases.

In some embodiments, the antibody is an anti-HER2 antibody with enhanced ADCC effect, which is used for targeting a cell or tissue of a patient to enhance the anti-tumor efficacy of the drug. In some embodiments, the antibody has a fucosylation level of no more than 10%. In some embodiments, the antibody has a fucosylation level of about 0, about 1%, about 2%, about 3%, about 6%, about 7%, about 8%, about 9%, about 10%, or a range between any two of these values (inclusive) or any value therein.

Provided in some embodiments is an antibody that specifically binds to HER2, and comprises: (a) an HCDR1 comprising a sequence set forth in SEQ ID NO: 1 or a sequence having 1 or 2 conservative amino acid substitutions as compared to the sequence set forth in SEQ ID NO: 1; (b) an HCDR2 comprising a sequence set forth in SEQ ID NO: 2 or a sequence having 1 or 2 conservative amino acid substitutions as compared to the sequence set forth in SEQ ID NO: 2; (c) an HCDR3 comprising a sequence set forth in SEQ ID NO: 3 or a sequence having 1 or 2 conservative amino acid substitutions as compared to the sequence set forth in SEQ ID NO: 3; (d) an LCDR1 comprising a sequence set forth in SEQ ID NO: 4 or a sequence having 1 or 2 conservative amino acid substitutions as compared to the sequence set forth in SEQ ID NO: 4; (e) an LCDR2 comprising a sequence set forth in SEQ ID NO: 5 or a sequence having 1 or 2 conservative amino acid substitutions as compared to the sequence set forth in SEQ ID NO: 5; and (f) an LCDR3 comprising a sequence set forth in SEQ ID NO: 6 or a sequence having 1 or 2 conservative amino acid substitutions as compared to the sequence set forth in SEQ ID NO: 6;
wherein the antibody has a fucosylation level of 0%-5%.

In some embodiments, the antibody comprises: (a) an HCDR1 comprising a sequence set forth in SEQ ID NO: 1, (b) an HCDR2 comprising a sequence set forth in SEQ ID NO: 2, (c) an HCDR3 comprising a sequence set forth in SEQ ID NO: 3, (d) an LCDR1 comprising a sequence set forth in SEQ ID NO: 4, (e) an LCDR2 comprising a sequence set forth in SEQ ID NO: 5, and (f) an LCDR3 comprising a sequence set forth in SEQ ID NO: 6.

In some embodiments, a heavy chain variable region of the antibody comprises a sequence set forth in SEQ ID NO: 7, or a sequence having at least 80% identity to the sequence set forth in SEQ ID NO: 7, or a sequence having one or more conservative amino acid substitutions as compared to the sequence set forth in SEQ ID NO: 7; and/or
a light chain variable region of the antibody comprises a sequence set forth in SEQ ID NO: 8, or a sequence having at least 80% identity to the sequence set forth in SEQ ID NO: 8, or a sequence having one or more conservative amino acid substitutions as compared to the sequence set forth in SEQ ID NO: 8.

In some embodiments, a heavy chain variable region of the antibody comprises a sequence set forth in SEQ ID NO: 7, and a light chain variable region of the antibody comprises a sequence set forth in SEQ ID NO: 8.

In some embodiments, a heavy chain of the antibody comprises a sequence set forth in SEQ ID NO: 9, or a sequence having at least 80% identity to the sequence set forth in SEQ ID NO: 9, or a sequence having one or more conservative amino acid substitutions as compared to the sequence set forth in SEQ ID NO: 9; and/or
a light chain of the antibody comprises a sequence set forth in SEQ ID NO: 10, or a sequence having at least 80% identity to the sequence set forth in SEQ ID NO: 10, or a sequence having one or more conservative amino acid substitutions as compared to the sequence set forth in SEQ ID NO: 10.

In some embodiments, a heavy chain of the antibody comprises a sequence set forth in SEQ ID NO: 9, and a light chain of the antibody comprises a sequence set forth in SEQ ID NO: 10.

In some embodiments, the antibody has a fucosylation level of about 0, about 1%, about 2%, about 3%, about 4%, about 5%, or a range between any two of these values (inclusive) or any value therein. In some embodiments, the antibody has a fucosylation level of 0. In some embodiments, the antibody does not bind to fucose.

In some embodiments, the antibody is a monoclonal antibody. In some embodiments, the antibody is a recombinant humanized monoclonal antibody. In some embodiments, the antibody is a recombinant humanized IgG1 monoclonal antibody.

In some embodiments, the antibody is expressed by a CHO cell, a HEK293F cell, a Cos1 cell, a Cos7 cell, a CV1 cell or a murine L cell. In some embodiments, the antibody is expressed by a CHO cell, a HEK293F cell, a Cos1 cell, a Cos7 cell, a CV1 cell or a murine L cell, in which an α-(1,6)-fucosyltransferase gene FUT8 is knocked out. In some embodiments, the antibody is expressed by a CHO cell in which an α-(1,6)-fucosyltransferase gene FUT8 is knocked out. The anti-HER2 antibody is purified by a conventional method, such as low-speed centrifugation to separate cells from the culture medium, high-speed centrifugation on the supernatant, and subsequent affinity purification of Protein A and ion exchange purification.

The present invention further provides an antibody that comprises: (a) an HCDR1 comprising a sequence set forth in SEQ ID NO: 1 or a sequence having 1 or 2 conservative amino acid substitutions as compared to the sequence set forth in SEQ ID NO: 1; (b) an HCDR2 comprising a sequence set forth in SEQ ID NO: 2 or a sequence having 1 or 2 conservative amino acid substitutions as compared to the sequence set forth in SEQ ID NO: 2; (c) an HCDR3 comprising a sequence set forth in SEQ ID NO: 3 or a sequence having 1 or 2 conservative amino acid substitutions as compared to the sequence set forth in SEQ ID NO: 3; (d) an LCDR1 comprising a sequence set forth in SEQ ID NO: 4 or a sequence having 1 or 2 conservative amino acid substitutions as compared to the sequence set forth in SEQ ID NO: 4; (e) an LCDR2 comprising a sequence set forth in SEQ ID NO: 5 or a sequence having 1 or 2 conservative amino acid substitutions as compared to the sequence set forth in SEQ ID NO: 5; and (f) an LCDR3 comprising a sequence set forth in SEQ ID NO: 6 or a sequence having 1 or 2 conservative amino acid substitutions as compared to the sequence set forth in SEQ ID NO: 6;
wherein the antibody comprises no less than 60% G0.

In some embodiments, the antibody comprises: (a) an HCDR1 comprising a sequence set forth in SEQ ID NO: 1, (b) an HCDR2 comprising a sequence set forth in SEQ ID NO: 2, (c) an HCDR3 comprising a sequence set forth in SEQ ID NO: 3, (d) an LCDR1 comprising a sequence set forth in SEQ ID NO: 4, (e) an LCDR2 comprising a sequence set forth in SEQ ID NO: 5, and (f) an LCDR3 comprising a sequence set forth in SEQ ID NO: 6.

In some embodiments, a heavy chain variable region of the antibody comprises a sequence set forth in SEQ ID NO: 7, or a sequence having at least 80% identity to the sequence set forth in SEQ ID NO: 7, or a sequence having one or more conservative amino acid substitutions as compared to the sequence set forth in SEQ ID NO: 7; and/or
a light chain variable region of the antibody comprises a sequence set forth in SEQ ID NO: 8, or a sequence having at least 80% identity to the sequence set forth in SEQ ID NO: 8, or a sequence having one or more conservative amino acid substitutions as compared to the sequence set forth in SEQ ID NO: 8.

In some embodiments, a heavy chain variable region of the antibody comprises a sequence set forth in SEQ ID NO: 7, and a light chain variable region of the antibody comprises a sequence set forth in SEQ ID NO: 8.

In some embodiments, a heavy chain of the antibody comprises a sequence set forth in SEQ ID NO: 9, or a sequence having at least 80% identity to the sequence set forth in SEQ ID NO: 9, or a sequence having one or more conservative amino acid substitutions as compared to the sequence set forth in SEQ ID NO: 9; and/or
a light chain of the antibody comprises a sequence set forth in SEQ ID NO: 10, or a sequence having at least 80% identity to the sequence set forth in SEQ ID NO: 10, or a sequence having one or more conservative amino acid substitutions as compared to the sequence set forth in SEQ ID NO: 10.

In some embodiments, a heavy chain of the antibody comprises a sequence set forth in SEQ ID NO: 9, and a light chain of the antibody comprises a sequence set forth in SEQ ID NO: 10.

In some embodiments, the antibody comprises no less than 60% G0. In some embodiments, the content of G0 glycoform is about 61%, about 62%, about 63%, about 65%, about 68%, about 71%, about 72%, about 73%, about 74%, about 75%, or a range between any two of these values (inclusive) or any value therein. In some embodiments, the content of G0 glycoform is 61%-75%. In some embodiments, the content of G0 glycoform is 72%-73%.

In some embodiments, the content of GO-GN glycoform is 4%-7%. In some embodiments, the content of GO-GN glycoform is about 4%, about 5%, about 6%, about 7%, or a range between any two of these values (inclusive) or any value therein. In some embodiments, the content of GO-GN glycoform is 5%-6%.

In some embodiments, the content of G1 glycoform is 8%-12%. In some embodiments, the content of G1 glycoform is about 8%, about 9%, about 10%, about 11%, about 12%, or a range between any two of these values (inclusive) or any value therein. In some embodiments, the content of G1 glycoform is 8%-9%.

In some embodiments, the content of G1' glycoform is 6%-12%. In some embodiments, the content of G1' glycoform is about 6%, about 7%, about 8%, about 9%, about 10%, about 11%, about 12%, or a range between any two of these values (inclusive) or any value therein. In some embodiments, the content of G1' glycoform is 8%-9%.

In some embodiments, the content of G2 glycoform is no more than 3%. In some embodiments, the content of G2 glycoform is no more than 2%, no more than 1%. In some embodiments, the content of G2 glycoform is about 0-1%.

In some embodiments, the content of G0 glycoform is 61%-75%, the content of GO-GN glycoform is 4%-7%, the content of G1' glycoform is 6%-12%, the content of G1 glycoform is 8%-12%, and the content of G2 glycoform is no more than 3%. In some embodiments, the content of G0 glycoform is about 72%-73%, the content of GO-GN glycoform is about 5%-6%, the content of G1 glycoform is 8%-9%, the content of G1' glycoform is 8%-9%, and the content of G2 glycoform is about 0-1%.

In some embodiments, the antibody is expressed by a CHO cell, a HEK293F cell, a Cos1 cell, a Cos7 cell, a CV1 cell or a murine L cell. In some embodiments, the antibody is expressed by a CHO cell, a HEK293F cell, a Cos1 cell, a Cos7 cell, a CV1 cell or a murine L cell, in which an α-(1,6)-fucosyltransferase gene FUT8 is knocked out. In some embodiments, the antibody is expressed by a CHO cell in which an α-(1,6)-fucosyltransferase gene FUT8 is knocked out. The anti-HER2 antibody is purified by a conventional method, such as low-speed centrifugation to separate cells from the culture medium, high-speed centrifugation on the supernatant, and subsequent affinity purification of Protein A and ion exchange purification.

The present invention further provides an antibody composition, wherein an antibody in the antibody composition specifically binds to HER2, and comprises: (a) an HCDR1 comprising a sequence set forth in SEQ ID NO: 1 or a sequence having 1 or 2 conservative amino acid substitutions as compared to the sequence set forth in SEQ ID NO: 1; (b) an HCDR2 comprising a sequence set forth in SEQ ID NO: 2 or a sequence having 1 or 2 conservative amino acid substitutions as compared to the sequence set forth in SEQ ID NO: 2; (c) an HCDR3 comprising a sequence set forth in SEQ ID NO: 3 or a sequence having 1 or 2 conservative amino acid substitutions as compared to the sequence set forth in SEQ ID NO: 3; (d) an LCDR1 comprising a sequence set forth in SEQ ID NO: 4 or a sequence having 1 or 2 conservative amino acid substitutions as compared to the sequence set forth in SEQ ID NO: 4; (e) an LCDR2 comprising a sequence set forth in SEQ ID NO: 5 or a sequence having 1 or 2 conservative amino acid substitutions as compared to the sequence set forth in SEQ ID NO: 5; and (f) an LCDR3 comprising a sequence set forth in SEQ ID NO: 6 or a sequence having 1 or 2 conservative amino acid substitutions as compared to the sequence set forth in SEQ ID NO: 6;
the content of main peak in the antibody composition is at least 72%.

In some embodiments, an antibody in the antibody composition comprises: (a) an HCDR1 comprising a sequence set forth in SEQ ID NO: 1, (b) an HCDR2 comprising a sequence set forth in SEQ ID NO: 2, (c) an HCDR3 comprising a sequence set forth in SEQ ID NO: 3, (d) an LCDR1 comprising a sequence set forth in SEQ ID NO: 4, (e) an LCDR2 comprising a sequence set forth in SEQ ID NO: 5, and (f) an LCDR3 comprising a sequence set forth in SEQ ID NO: 6.

In some embodiments, a heavy chain variable region of the antibody comprises a sequence set forth in SEQ ID NO: 7, or a sequence having at least 80% identity to the sequence set forth in SEQ ID NO: 7, or a sequence having one or more conservative amino acid substitutions as compared to the sequence set forth in SEQ ID NO: 7; and/or
a light chain variable region of the antibody comprises a sequence set forth in SEQ ID NO: 8, or a sequence having at least 80% identity to the sequence set forth in SEQ ID NO: 8, or a sequence having one or more conservative amino acid substitutions as compared to the sequence set forth in SEQ ID NO: 8.

In some embodiments, a heavy chain variable region of the antibody comprises a sequence set forth in SEQ ID NO: 7, and a light chain variable region of the antibody comprises a sequence set forth in SEQ ID NO: 8.

In some embodiments, a heavy chain of the antibody comprises a sequence set forth in SEQ ID NO: 9, or a sequence having at least 80% identity to the sequence set forth in SEQ ID NO: 9, or a sequence having one or more conservative amino acid substitutions as compared to the sequence set forth in SEQ ID NO: 9; and/or
a light chain of the antibody comprises a sequence set forth in SEQ ID NO: 10, or a sequence having at least 80% identity to the sequence set forth in SEQ ID NO: 10, or a sequence having one or more conservative amino acid substitutions as compared to the sequence set forth in SEQ ID NO: 10.

In some embodiments, a heavy chain of the antibody comprises a sequence set forth in SEQ ID NO: 9, and a light chain of the antibody comprises a sequence set forth in SEQ ID NO: 10.

In some embodiments, the content of main peak in the antibody composition is 73%-82%. In some embodiments, the content of main peak in the antibody composition is about 73%, about 74%, about 75%, about 76%, about 77%, about 78%, about 79%, about 80%, about 81%, about 82%, or a range between any two of these values (inclusive) or any value therein. In some embodiments, the content of main peak in the antibody composition is 77%-81%.

In some embodiments, the content of a basic variant in the antibody composition is no more than 8% or 6%. In some embodiments, the content of a basic variant in the antibody composition is about 1%, about 2%, about 3%, about 4%, about 5%, about 6%, about 7%, about 8%, or a range between any two of these values (inclusive) or any value therein. In some embodiments, the content of a basic variant in the antibody composition is 1%-6%. In some embodiments, the content of a basic variant in the antibody composition is 2%-5%. In some embodiments, the content of a basic variant in the antibody composition is 1%-4%.

In some embodiments, the antibody is expressed by a CHO cell, a HEK293F cell, a Cos1 cell, a Cos7 cell, a CV1 cell or a murine L cell. In some embodiments, the antibody is expressed by a CHO cell, a HEK293F cell, a Cos1 cell, a Cos7 cell, a CV1 cell or a murine L cell, in which an α-(1,6)-fucosyltransferase is knocked out. In some embodiments, the antibody is expressed by a CHO cell in which an α-(1,6)-fucosyltransferase is knocked out. The anti-HER2 antibody is purified by a conventional method, such as low-speed centrifugation to separate cells from the culture medium, high-speed centrifugation on the supernatant, and subsequent affinity purification of Protein A and ion exchange purification.

In some embodiments, the antibody is BAT0303F, a recombinant humanized anti-HER-2 monoclonal antibody that is glycosylated, and has an antibody sequence identical to that of pertuzumab; in addition, the Antibody BAT0303F is expressed by a CHO cell with fucose knocked out, and has enhanced ADCC effect and further improves efficacy.

In some embodiments, the antibody is an isolated anti-HER2 antibody.

The present invention further provides a nucleic acid encoding the antibody. In some embodiments, the nucleic acid molecule is an isolated nucleic acid.

The present invention further provides a vector comprising the nucleic acid. In some embodiments, the vector is an isolated vector.

The present invention further provides a host cell comprising the nucleic acid molecule or the vector. In some embodiments, the host cell is an isolated host cell. In some embodiments, the host cell is a CHO cell, a HEK293F cell, a Cos1 cell, a Cos7 cell, a CV1 cell or a murine L cell.

The present invention further provides a treatment method and use. In some embodiments, provided is a method for treating or ameliorating a cell proliferative disease, wherein the method comprises administering to a patient an effective amount of the antibody. In some embodiments, provided is use of the antibody, the nucleic acid molecule, the vector or the host cell in the manufacture of a medicament for treating or ameliorating a cell proliferative disease.

The present invention further provides a diagnostic method and use. In some embodiments, provided is a method for detecting an amount of HER2 expression in a sample, wherein the method comprises contacting the sample with the antibody, such that the antibody binds to HER2, and detecting the binding, i.e., an amount of HER2 protein in the sample. In some embodiments, provided is use of the antibody in the manufacture of a kit for the diagnosis of a cell proliferative disease. In some embodiments, provided is a diagnostic kit comprising the antibody.

The present invention further provides a pharmaceutical composition comprising the antibody or the antibody composition, and a pharmaceutically acceptable carrier.

### BRIEF DESCRIPTION OF THE DRAWINGS

FIG. 1 is a profile of pHC plasmid in Example 2; wherein intron A represents interferon α-2b gene, SV40E and hCMV represents promoters, polyA represents poly tail, heavy chain Gene represents a heavy chain gene, GS DNA represents glutamine synthetase gene, and beta-lactamse represents ampicillin screening label gene.
FIG. 2 is a profile of pLC plasmid in Example 2; wherein light chain Gene represents a light chain gene.
FIG. 3 shows the binding activity of the anti-HER2 antibody to HER2 antigen in Example 3; wherein control represents Perjeta^{®}.
FIG. 4 is a graph showing the inhibitory effects of the anti-HER2 antibody on tumor cell proliferation in Example 8; wherein the abscissa represents concentration.
FIG. 5 shows the ADCC effect of anti-HER2 antibody on HCC1954 cells in Example 9.
FIG. 6 shows the ADCC effect of anti-HER2 antibody on NCI-N87 cells in Example 9; wherein the abscissa represents concentration.
FIG. 7 shows that all anti-HER2 antibody in Example 10 can inhibit the growth of NCI-N87.
FIG. 8 shows the effect of anti-HER2 antibody in Example 12 on body weight of tumor-bearing mice.

### Definitions

Unless otherwise specified, each of the following terms shall have the meaning described below.

It should be noted that the term "an" entity refers to one or more of the entities. For example, "an antibody" should be interpreted as one or more antibodies. As such, the terms "a" (or "an"), "one or more" and "at least one" can be used interchangeably herein.

As used herein, the terms "containing" or "comprising" mean that the compositions, methods and the like comprise the recited elements, such as components or steps and do not exclude the others. "Consisting essentially of..." means that the compositions and methods exclude other elements that have a material affect on the characteristics of the combination, but does not exclude elements that do not materially affect the characteristics of the compositions or methods. "Consisting of" shall mean excluding elements not specifically recited.

The term "polypeptide" is intended to encompass both the singular form "polypeptide" and the plural form "polypeptides", and refers to a molecule consisting of amino acid monomer linearly linked by amide bonds (or peptide bonds). The term "polypeptide" refers to any single chain or multiple chains of two or more amino acids and does not refer to a particular length of the product. Thus, included within the definition of "polypeptide" are peptides, dipeptides, tripeptides, oligopeptides, "proteins", "amino acid chains", or any other term used to refer to chains of two or more amino acids, and the term "polypeptide" may be used in place of, or interchangeably with, any of the above terms. The term "polypeptide" is also intended to refer to a product of post-expression polypeptide modification, including but not limited to glycosylation, acetylation, phosphorylation, amidation, derivatization by known protecting/blocking groups, proteolytic cleavage or non-naturally occurring amino acid modification. The polypeptide may be derived from a natural biological source or produced by recombinant techniques, but it is not necessarily translated from a specified nucleic acid sequence, and it may be produced in any manner, including chemical synthesis.

"Amino acid" refers to an organic compound containing both an amino group and a carboxyl group, such as an α-amino acid that can be encoded by a nucleic acid, either directly or in the form of a precursor. A single amino acid is encoded by a nucleic acid consisting of three nucleotides (so-called codon or base triplet). Each amino acid is encoded by at least one codon. An amino acid may be encoded by different codons, which is known as "codon degeneracy". Amino acids include natural amino acids and non-natural amino acids. Natural amino acids include alanine (three-letter code: ala, one-letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

"Conservative amino acid substitution" refers to the replacement of one amino acid residue with another amino acid residue having a side chain (R group) of similar chemical nature (e.g., charge or hydrophobicity). Generally, conservative amino acid substitutions do not substantially alter the functional properties of the protein. Examples of amino acid classes with a side chain of similar chemical nature include: 1) an aliphatic side chain: glycine, alanine, valine, leucine and isoleucine; 2) an aliphatic hydroxyl side chain: serine and threonine; 3) an amide-containing side chain: asparagine and glutamine; 4) an aromatic side chain: phenylalanine, tyrosine and tryptophan; 5) a basic side chain: lysine, arginine and histidine; and 6) an acidic side chain: aspartic acid and glutamic acid. The number of amino acids of "conservative amino acid substitutions of a light chain variable region, a heavy chain variable region, a light chain and a heavy chain" in the present invention is about 1, about 2, about 3, about 4, about 7, about 9, about 11, about 20, about 22, about 24, about 28, about 31, about 33, about 36, about 39, about 43, about 45, or a range between any two of these values (inclusive) or any value therein.

The term "isolated" as used herein with respect to cells, nucleic acids, polypeptides, antibodies and the like, e.g., "isolated" DNA, RNA, polypeptides, antibodies, refers to molecules that are separated from one or more other components, e.g., DNA or RNA, in the natural environment of the cell. The term "isolated" as used herein also refers to nucleic acids or peptides that are substantially free of cellular materials, viral materials or cell culture media when produced by recombinant DNA techniques, or of chemical precursors or other chemicals when chemically synthesized. In addition, "isolated nucleic acid" is intended to include nucleic acid fragments that do not and will not occur in nature. The term "isolated" is also used herein to refer to cells or polypeptides that are separated from other cellular proteins or tissues. Isolated polypeptides are intended to include both purified and recombinant polypeptides. Isolated polypeptides, antibodies and the like are usually prepared by at least one purification step. In some embodiments, the purity of the isolated nucleic acids, polypeptides and antibodies is at least about 50%, about 60%, about 70%, about 80%, about 90%, about 95%, about 99%, or a range between any two of these values (inclusive) or any value therein.

The term "recombinant", with regard to a polypeptide or polynucleotide, is intended to refer to a polypeptide or polynucleotide that does not occur in nature, and non-limiting examples can be combined to produce a polynucleotide or polypeptide that does not normally occur in nature.

"Homology", "identity" or "similarity" refers to sequence similarity between two peptides or between two nucleic acid molecules. Homology can be determined by comparing the positions that can be aligned in the sequences. When a position of the compared sequences is occupied by the same base or amino acid, then the molecules are homologous at that position. The degree of homology between the sequences is a function of the number of matching or homologous positions shared by the sequences.

In some embodiments, at least 80% identity is about 80% identity, about 81% identity, about 82% identity, about 83% identity, about 85% identity, about 87% identity, about 89% identity, about 90% identity, about 91% identity, about 92% identity, about 93% identity, about 94% identity, about 95% identity, about 96% identity, about 97% identity, about 98% identity, about 99% identity, or a range between any two of these values (inclusive) or any value therein.

A polynucleotide and a polynucleotide sequence (or polypeptide or antibody sequence) having a certain percentage (e.g., 90%, 95%, 98% or 99%) of "identity or sequence identity" to another sequence refers to that when the sequences are aligned, the percentage of bases (or amino acids) in the sequence are the same. This alignment and identity percentage, or sequence identity can be determined using visual inspection or software programs known in the art, such as the software programs described in Ausubel et al. eds., (2007), Current Protocols in Molecular Biology*.* Preferably, the alignment is performed using default parameters. One alignment program is BLAST using default parameters, such as BLASTN and BLASTP, both using the following default parameters: Genetic code = standard; filter = none; strand = both; cutoff = 60; expect = 10; Matrix = BLOSUM62; Descriptions = 50 sequences; sort by = HIGH SCORE; Databases = non-redundant; GenBank + EMBL + DDBJ + PDB + GenBank CDS translations + SwissProtein + SPupdate + PIR. Biologically equivalent polynucleotides are polynucleotides having the above specified percentage identity and encoding polypeptides having identical or similar biological activity.

A polynucleotide consists of a specific sequence of four nucleotide bases: adenine (A), cytosine (C), guanine (G), thymine (T)/uracil (U, instead of thymine when the polynucleotide is RNA). A "polynucleotide sequence" can be a letter representation of a polynucleotide molecule. The letter representation can be input into a database in a computer with a central processing unit and used for bioinformatics applications, such as for functional genomics and homology searches.

The terms "polynucleotide" and "oligonucleotide" are used interchangeably to refer to a polymeric form of nucleotides of any length, whether deoxyribonucleotides or ribonucleotides or analogs thereof. The polynucleotide may have any three-dimensional structure and may perform any function, known or unknown. The following are non-limiting examples of polynucleotides: genes or gene fragments (such as probes, primers, EST or SAGE tags), exons, introns, messenger RNA (mRNA), transport RNA, ribosomal RNA, ribozymes, cDNA, dsRNA, siRNA, miRNA, recombinant polynucleotides, branched polynucleotides, plasmids, vectors, isolated DNA of any sequence, isolated RNA of any sequence, nucleic acid probes and primers. Polynucleotides can include modified nucleotides, such as methylated nucleotides and nucleotide analogs. If present, structural modifications to the nucleotide can be made before or after the assembly of the polynucleotide. The sequence of nucleotides can be interrupted by non-nucleotide components. The polynucleotide may be further modified after polymerization, such as by conjugation with a labeling component. This term also refers to double-stranded and single-stranded molecules. Unless otherwise stated or required, examples of any polynucleotide disclosed herein include a double-stranded form and each of the two complementary single-stranded forms known or predicted to constitute the double-stranded form.

The term "encoding" as applied to a polynucleotide refers to a polynucleotide known to "encode" a polypeptide. When in its native state or manipulated by methods well known to those skilled in the art, the polypeptide and a fragment thereof can be produced by transcript and/or translation.

"Antibody" refers to a polypeptide or polypeptide complex that specifically recognizes and binds to an antigen. The antibody may be an intact antibody and any antigen-binding fragment or single chain thereof. The term "antibody" thus includes any protein or peptide comprising at least a portion of an immunoglobulin molecule that has biological activity for binding to an antigen. The antibody and the antigen-binding fragment include, but are not limited to, complementarity determining regions (CDRs) of a heavy or light chain or a ligand binding portion thereof, heavy chain variable regions (VHs), light chain variable regions (VLs), heavy chain constant regions (CHs), light chain constant regions (CLs), framework regions (FRs) or any portion thereof, or at least a portion of a binding protein. The CDRs include light chain CDRs (LCDR1-3) and heavy chain CDRs (HCDR1-3).

A "monoclonal antibody" (mAb) is an antibody prepared from the same immune cell which is all clones of a single parent cell. The monoclonal antibody can have monovalent affinity because it binds to the same epitope (the antigen portion that the antibody recognizes). In contrast, a polyclonal antibody binds to multiple epitopes, and are usually secreted by several different plasma cells. The monoclonal antibody can be prepared by hybridoma, recombinant, transgene technology, or other technologies known to those skilled in the art.

The term "antibody" includes a wide variety of polypeptides that can be biochemically distinguished. Those skilled in the art will appreciate that the classes of heavy chains include gamma, mu, alpha, delta, or epsilon (γ, µ, α, δ, or ε), and some subclasses (e.g., γ1-γ4). The nature of this chain determines the "type" of the antibody as IgG, IgM, IgA, IgD or IgE. Immunoglobulin subclasses (isotypes), such as IgG1, IgG2, IgG3, IgG4, IgG5, etc., have been well characterized and the functional specificity imparted is also known. All types of immunoglobulins are within the scope of the present invention. In some embodiments, the immunoglobulin molecule is an IgG species. IgG typically contains two identical light chain polypeptides with a molecular weight of about 23,000 Daltons and two identical heavy chain polypeptides with a molecular weight of about 53,000-70,000. These four chains are connected in a "Y" configuration through disulfide bonds, wherein the light chain starts at the opening of "Y" configuration and extends through the variable region to surround the heavy chain.

Light chains can be classified into kappa (κ) or lambda (λ). Each heavy chain may be connected with a κ or λ light chain. In general, when an immunoglobulin is produced by a hybridoma, a B cell or a genetically engineered host cell, the light and heavy chains are bound by covalent bonds, and the "tail" portions of the two heavy chains are bound by covalent disulfide bonds or non-covalent bonds. In the heavy chains, the amino acid sequence extends from the N terminus at the forked end of the Y configuration to the C terminus at the bottom of each chain. The immunoglobulin κ light chain variable region is Vκ; the immunoglobulin λ light chain variable region is V_{λ}.

Both the light and heavy chains are divided into structurally and functionally homologous regions. The terms "constant" and "variable" are used in accordance with function. The light chain variable region (VL) and heavy chain variable region (VH) determine the antigen recognition and specificity. The light chain constant region and the heavy chain constant region impart important biological properties such as secretion, transplacental movement, Fc receptor binding, and complement binding. By convention, the numbering of amino acids in the constant regions increases as they become further away from the antigen-binding site of the antibody or amino terminus. The N-terminal portion is the variable region, and the C-terminal portion is the constant region; the CH3 and CL domains comprise the carboxyl termini of the heavy chain and light chain, respectively.

In naturally occurring antibodies, the six "complementarity determining regions" or "CDRs" present in each antigen-binding domain are short, non-contiguous, antigen-specific binding amino acid sequences that form the antigen-binding domain, assuming that the antibody is present in its three-dimensional configuration in an aqueous environment. The remaining amino acids in the antigen-binding domain, referred to as the "framework" region, exhibit little intermolecular variability. Most of the framework regions adopt a β-sheet conformation, with the CDRs forming a loop structure connected to, or in some cases forming part of, the β-sheet structure. Thus, the framework regions position the CDRs in a correct orientation by interchain non-covalent interactions through forming a scaffold. The antigen-binding domain with the specifically positioned CDRs forms a surface complementary to an epitope on the antigen that facilitates non-covalent binding of the antibody to its antigenic epitope. For given heavy or light chain variable region, amino acids comprising the CDRs and the framework regions may be identified by one of ordinary skills in the art according to known method (see, e.g., Kabat, E., et al., U.S. Department of Health and Human Services, Sequences of Proteins of Immunological Interest, (1983) and Chothia and Lesk, J. Mol. Biol., 196:901-917 (1987)).

Where the terms used and/or accepted in the art have two or more definitions, the definitions of the terms used herein include all of these meanings unless explicitly indicated as opposite. One specific example is the use of the term "complementarity determining regions" ("CDRs") to describe non-contiguous antigen-binding sites found within the variable regions of heavy and light chain polypeptides. This specific region is described in Kabat et al., U.S. Dept. of Health and Human Services, Sequences of Proteins of Immunological Interest (1983) and Chothiaet al., J. Mol. Biol. 196:901-917 (1987), which is incorporated herein by reference in its entirety.

CDRs defined according to Kabat and Chothia systems include overlaps or subsets of amino acid residues when compared to each other. Nevertheless, it is within the scope of the present invention to apply any definition to refer to the CDRs of an antibody or a variant thereof. The exact number of residues comprising a specific CDR will vary according to the sequence and size of the CDR. Those skilled in the art can generally determine which specific residues a CDR comprises based on the variable region amino acid sequence of an antibody.

Kabat et al. also defines a numbering system applicable to the variable region sequence of any antibody. One of ordinary skill in the art can apply the "Kabat numbering" system to any variable region sequence without depending on other experimental data beyond the sequence itself. "Kabat numbering" refers to the numbering system proposed by Kabat et al., U.S. Dept. of Health and Human Services in "Sequence of Proteins of Immunological Interest" (1983). EU numbering system can be also applicable to the antibody.

"Light chain constant region" includes a part of amino acid sequence from the light chain of an antibody. Preferably, the light chain constant region (CL) comprises at least one of a constant κ domain or a constant λ domain. A "light chain-heavy chain pair" refers to a collection of light and heavy chains that can form dimers through disulfide bonds between the CL domain of the light chain and the CH1 domain of the heavy chain.

"VH domain" includes the variable domain at the amino terminus of an immunoglobulin heavy chain, and "CH1 domain" includes the first constant region (mostly at the amino terminus) of an immunoglobulin heavy chain. The CH2 domain is not closely paired with other domains, but rather two N-linked branched carbohydrate chains are inserted between the two CH2 domains of an intact native IgG molecule. It is also documented that the CH3 domain extends from the CH2 domain to the C terminus of the IgG molecule, and comprises about 108 residues. "Hinge region" includes a portion of the heavy chain region connecting the CH1 domain and CH2 domain. The hinge region comprises about 25 residues and is flexible, thereby enabling independent movement of the two N-terminal antigen-binding regions. The hinge region can be subdivided into three distinct domains: upper, middle and lower hinge domains (Roux et al., J. Immunol., 161:4083 (1998)).

"Disulfide bond" refers to a covalent bond formed between two sulfur atoms. The thiol group of cysteine can form a disulfide bond or a bridge with a second thiol group. In most naturally occurring IgG molecules, the CH1 and CL regions are linked by a disulfide bond.

"Specifically bind to" or "specific for" generally refers to that an antibody or an antigen-binding fragment and a specific antigen bind to an epitope through its antigen-binding domain to form a relatively stable complex. "Specificity" can be expressed by relative affinity of an antibody or an antigen-binding fragment for binding to a specific antigen or epitope. For example, an antibody "A" can be considered to have a higher specificity for an antigen than an antibody "B" if the antibody "A" has a greater relative affinity for the antigen than the antibody "B". Specific binding can be described by the equilibrium dissociation constant (KD). A smaller KD means a tighter binding. Methods for determining whether two molecules specifically bind to each other are well known in the art, and include, for example, equilibrium dialysis, surface plasmon resonance, biofilm layer interferometry, and the like. Antibodies that "specifically bind" to antigen a include antibodies that have an equilibrium dissociation constant KD of less than or equal to about 100 nM, less than or equal to about 10 nM, less than or equal to about 5 nM, or less than or equal to about 1 nM with antigen a.

"Antibody-dependent cell-mediated cytotoxicity" (ADCC) is a mechanism of cell-mediated immune defense in which the membrane surface receptor of the effector cell specifically binds to the antibody to actively lyse target cells. ADCC is a part of the humoral immune response, and can act to limit and contain infection.

ADCC requires an effector cell, typically a natural killer (NK) cell that interacts with the IgG antibody. However, macrophages, neutrophils and eosinophils can also mediate ADCC, such as eosinophils killing certain parasitic worms known as helminths via the IgE antibody. In addition, ADCC is part of the adaptive immune response because it is dependent on previous antibody responses.

An "Fc region" is the tail region of an antibody which interacts with cell surface receptors and some proteins of the complement system. This property allows the antibody to activate the immune system. In the IgG, IgA and IgD antibody isotypes, the Fc region consists of two identical protein fragments, and is derived from a second and third constant regions of the two heavy chains of the antibody; in the IgM and IgE antibody isotypes, the Fc region contains three heavy chain constant domains (CH2-4); and the Fc region of IgG has highly conservative N-glycosylation sites. Glycosylation of the Fc fragment is essential for Fc receptor-mediated activity, and different glycoforms have different effects on the pharmaceutical properties of the therapeutic antibody.

An "Fc receptor" or "FcR" is the protein found on the surface of certain cells including B lymphocytes, follicular dendritic cells, natural killer cells, macrophages, neutrophils, eosinophils, basophils, human platelets, mast cells and the like, which all contribute to the protection of immune system function. The name Fc receptor derives from its binding specificity to the Fc region of an antibody. The Fc receptors bind to antibodies that attaches to infected cells or invading pathogens. The activity of the Fc receptors is exerted by destruction of the microorganism by phagocytic and cytotoxic cells, or by de-infection of the cells by antibody-mediated phagocytosis, or by antibody-dependent cell-mediated cytotoxicity. Some viruses, such as flaviviruses, use Fc receptors to help them infect cells through a mechanism known as antibody-dependent infection enhancement.

Several different types of Fc receptors are classified based on the type of antibody they recognize. For example, those binding to the antibody IgG are referred to as Fc-γ receptors (FcyRs), those binding to IgA are referred to as Fc-α receptors (FcαRs), and those binding to IgE are referred to as Fc-ε receptors (FcεRs). The class of FcRs may also be distinguished by the signaling properties of the cells (macrophages, granulocytes, natural killer cells, T cells and B cells) expressing the FcRs and of each receptor.

All Fcγ receptors (FcyRs) belong to the immunoglobulin superfamily and are the most important Fc receptors for inducing phagocytosis in microorganisms. This family includes several members, FcyRI (CD64), FcγRIIA (CD32), FcyRIIB (CD32), FcγRIIIA (CD16a), FcγRIIIB (CD16b), which differ in antibody affinity due to their different molecular structures. For example, FcyRI has a greater binding ability to IgG than FcγRII or FcyRIII. FcyRI also has an extracellular portion consisting of three immunoglobulin (Ig)-like domains, and has more domains than FcyRII or FcyRIII. This property allows FcyRI to bind to the only IgG molecule (or monomer), while all Fcγ receptors must bind to multiple IgG molecules in an immune complex to be activated. Fcγ receptors differ in their affinity for IgG, and different IgG subclasses have unique affinity for each Fcγ receptor. These interactions are further regulated by glycans (oligosaccharides) at position CH2-84.4 of IgG. For example, fucose containing glycans at the position CH2-84.4 reduces IgG affinity for FcγRIIIA by generating steric hindrance.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of extent of disease, stabilized (i.e., not worsening) state of disease, delay or slowing of disease progression, amelioration, palliation, alleviation, or elimination (whether partial or total), prolongation of life expectancy in the absence of treatment, and the like, as either detectable or undetectable. Patients in need of treatment include patients already with a condition or disorder, patients susceptible to a condition or disorder, or patients in need of prevention of the condition or disorder, patients who may or are expected to benefit from administration of the antibody or the pharmaceutical composition disclosed herein for detection, diagnostic procedures, and/or treatment.

"Patient" refers to any mammal in need of diagnosis, prognosis or treatment, including humans, dogs, cats, rabbits, mice, horses, cattle and the like.

"About" refers to a general error range for corresponding values as readily understood by those skilled in the relevant art. In some embodiments, "about" mentioned herein refers to the values described and ranges thereof of ±10%, ±5%, or ±1%.

The monoclonal antibodies can be separated based on the charge they carry and charge heterogeneity can be analyzed, and common used analytical methods are isoelectric focusing (IEF) gel electrophoresis, capillary isoelectric focusing (cIEF) gel electrophoresis, cation exchange (CEX) chromatography and anion exchange (AEX) chromatography. These variants are generally referred to as acidic variants and basic variants, i.e., acid peaks and basic peaks, as compared to the main peak. When analyzed by CEX, the acid peak elutes earlier than the main peak, while the basic peak elutes later than the main peak.

### Anti-HER2 antibody

The present invention provides an anti-HER2 antibody, wherein the test antibody exhibits potent binding activity, biological activity (enhanced ADCC effect), and may be useful for therapeutic and diagnostic uses.

In some embodiments, a heavy chain of the antibody comprises a sequence set forth in SEQ ID NO: 9, and a light chain of the antibody comprises a sequence set forth in SEQ ID NO: 10. In some embodiments, the antibody is a monoclonal antibody.

In some embodiments, the antibody is a monoclonal antibody, and the antibody has a fucosylation level of 0%-5%. In some embodiments, the antibody is a monoclonal antibody, and the antibody has a fucosylation level of 0.

In some embodiments, the antibody is a monoclonal antibody having one or more of the following characteristics: the content of G0 glycoform being 61%-75%, the content of GO-GN glycoform being 4%-7%, the content of G1' glycoform being 6%-12%, the content of G1 glycoform being 8%-12%, and the content of G2 glycoform being no more than 3%. In some embodiments, the content of G0 glycoform is about 72%-73%, the content of GO-GN glycoform is about 5%-6%, the content of G1' glycoform is 8%-9%, the content of G1 glycoform is 8%-9%, and the content of G2 glycoform is about 0-1%.

In some embodiments, the antibody is a monoclonal antibody, and the content of main peak in the antibody composition is at least 72%, such as 73%-82%. In some embodiments, the antibody is a monoclonal antibody, and the content of a basic variant in the antibody composition is no more than 8% or 6%. In some embodiments, the antibody is a monoclonal antibody, the content of main peak in the antibody composition is 77%-81%, and the content of a basic variant in the antibody composition is 1%-6%. In some embodiments, the antibody is a monoclonal antibody, the content of main peak in the antibody composition is 77%-78%, and the content of a basic variant in the antibody composition is 2%-5%. In some embodiments, the antibody is a monoclonal antibody, the content of main peak in the antibody composition is 79%-81%, and the content of a basic variant in the antibody composition is 1%-4%.

In some embodiments, the antibody is expressed by a CHO cell, a HEK293F cell, a Cos1 cell, a Cos7 cell, a CV1 cell or a murine L cell, in which an α-(1,6)-fucosyltransferase gene FUT8 is knocked out. In some embodiments, the antibody is expressed by a CHO cell in which an α-(1,6)-fucosyltransferase gene FUT8 is knocked out.

It will also be appreciated by those of ordinary skills in the art that the antibody disclosed herein may be substituted to have an amino acid sequence that differs from the naturally occurring amino acid sequence of the antibody. For example, the substituted amino acid sequence can be similar to the starting sequence, for example, having a proportion of identity to the starting sequence, such as about 80%, about 85%, about 90%, about 95%, about 98% or about 99% identical to the starting sequence, or a range between any two of these values (inclusive) or any value therein.

In certain embodiments, an amino acid sequence comprised in an antibody has one or more modification groups. For example, the anti-HER2 antibody disclosed herein may comprise a flexible linker sequence, or may be modified to add a functional group (e.g., PEG, a drug, a toxin, or a tag).

The antibodies disclosed herein include modified derivatives, i.e., modified by covalent attachment of any type of molecule to the antibody, wherein the covalent attachment does not prevent the antibody from binding to the antigenic epitope. The following examples are included but not by way of limitation, wherein an antibody may be modified by glycosylation, acetylation, pegylation, phosphorylation, amidation, derivatization by known protecting/blocking groups, proteolytic cleavage, attachment to cellular ligands or other proteins, or the like. Any of a number of chemical modifications may be made by techniques in the prior art, including but not limited to specific chemical cleavage, acetylation, formylation, metabolic synthesis of tunicamycin, and the like.

In some embodiments, the antibody may be conjugated with a therapeutic agent, a prodrug, a peptide, a protein, an enzyme, a virus, a lipid, a biological response modifier, an agent, or PEG.

The antibody may be conjugated or fused to a therapeutic agent, which includes a detectable label (such as a radiolabel), an immunomodulator, a hormone, an enzyme, an oligonucleotide, a photoactive therapeutic agent, a cytotoxic agent, an ultrasound enhancing agent, a nonradioactive label and a composition thereof, and other such agents known in the art.

The antibody may be detectably labeled by coupling to a chemiluminescent compound. The presence of the chemiluminescent-labeled antibody is then determined by detecting the luminescence that occurs during the course of chemical reactions. Examples of chemiluminescent-labeled compounds include luminol, isoluminol, aromatic acridinium esters, imidazole, acridinium salts and oxalate esters.

### Methods for Preparing Antibodies and Antibody-Encoding Polynucleotides

The present invention further discloses a polynucleotide or a nucleic acid molecule encoding the antibody and a derivative thereof of the present invention. The polynucleotide disclosed herein may encode a heavy chain variable region, a light chain variable region, an Fc region, a portion of a heavy chain variable region or a portion of a light chain variable region. Methods of making antibodies are well known in the art and are described herein. In certain embodiments, the variable and constant regions of the antibody disclosed herein are of fully human. The fully human antibody can be prepared using techniques known in the art and disclosed herein. For example, the fully human antibody against a specific antigen can be prepared by administering the antigen to transgenic animals that have been modified to produce the fully human antibody in response to antigen challenge. Exemplary techniques that can be used to prepare such an antibody are found in U.S. Patent Nos. 6,458,592 and 6,420,140; which are incorporated herein by reference in their entireties.

The binding specificity of the anti-HER2 antibody disclosed herein can be detected by an *in vitro* assay, such as co-immunoprecipitation, radioimmunoassay (RIA) or enzyme-linked immunosorbent assay (ELISA).

In addition, using conventional recombinant DNA techniques, one or more CDRs of the antibody disclosed herein can be inserted into framework regions, e.g., into human framework regions to construct a humanized non-fully human antibody. The framework regions may be naturally occurring or consensus framework regions, preferably human framework regions (see Chothia et al., J. Mol. Biol. 278:457-479 (1998), in which a range of human framework regions are listed). Some polynucleotides may encode antibodies produced by the framework region and CDR combinations that specifically bind to at least one epitope of an antigen of interest. One or more amino acid substitutions may be made within the framework regions, and amino acid substitutions capable of improving the binding of the antibody to the antigen thereof may be selected. Additionally, substitution or deletion of one or more cysteine residues in the variable regions involved in interchain disulfide bond formation can be made in this manner, thereby producing an antibody molecule lacking one or more interchain disulfide bonds. Other alterations to the polynucleotides made within the technology scope of the art are also encompassed by the present invention.

Antibodies can be prepared by using conventional recombinant DNA techniques. Antibody-producing cell lines can be selected, constructed and cultured using techniques well known to those skilled in the art. These techniques are described in various laboratory manuals and main publications. In this aspect, for the techniques suitable for use in the present invention described below, reference is made to Current Protocols in Immunology, Coligan et al., Eds., Green Publishing Associates and Wiley-Interscience, John Wiley and Sons, New York (1991), Recombinant DNA Technology for Production of Protein Therapeutics in Cultured Mammalian Cells, D.L. Hacker, F.M. Wurm, Reference Module in Life Sciences, 2017; which is incorporated herein by reference in its entirety, including the supplements.

In some embodiments, the DNA encoding the antibody can be designed and synthesized according to the antibody amino acid sequence described herein by conventional methods, inserted into an expression vector, and transfected into a host cell. The transfected host cell is then cultured in a medium to produce the monoclonal antibody. In some embodiments, the vector expressing the antibody comprises at least one promoter element, an antibody coding sequence, a transcription termination signal and a poly-A tail. Other elements include enhancers, Kozak sequences, and donor and recipient sites flanking the inserted sequence for RNA splicing. Efficient transcription can be obtained by early and late promoters of SV40, long terminal repeats from retroviruses such as RSV, HTLV1 and HIVI, and early promoters of cytomegalovirus, and promoters from other cells such as actin promoter can also be used. Suitable expression vectors may include pIRES1neo, pRetro-Off, pRetro-On, PLXSN, or Plncx, pcDNA3.1 (+/-), pcDNA/Zeo (+/-), pcDNA3.1/Hygro(+/-), PSVL, PMSG, pRSVcat, pSV2dhfr, pBC12MI, pCS2, etc. Commonly used mammalian cells include 293 cells, Cos1 cells, Cos7 cells, CV1 cells, murine L cells, CHO cells and the like.

In some embodiments, the inserted gene fragment should comprise a screening label, common ones of which include screening genes such as dihydrofolate reductase, glutamine synthetase, neomycin resistance and hygromycin resistance, to facilitate the screening and separation of cells that have been successfully transfected. The constructed plasmid is transfected to a host cell without the above genes, and the successfully transfected cells are cultured in a large quantity in a selective culture medium to produce the desired target protein.

In some embodiments, the substitutions described herein are conservative amino acid substitutions.

### Therapeutic Method

The present invention further provides a treatment method and use. In some embodiments, provided is a method for treating a cell proliferative disease, wherein the method comprises administering to a patient an effective amount of the anti-HER2 antibody. In some embodiments, provided is use of the anti-HER2 antibody in the treatment of a cell proliferative disease. In some embodiments, provided is use of the anti-HER2 antibody in the manufacture of a medicament for treating a cell proliferative disease.

In some embodiments, the cell proliferative disease is cancer. In some embodiments, the cancer is a HER2-positive cancer. In some embodiments, the cancer includes, but is not limited to, lymphoma, blastoma, sarcoma, leukemia and lymphoid malignancies. More specific examples of such cancers include, but are not limited to, squamous cell carcinoma (e.g.,epithelial squamous cell carcinoma), lung cancer (including small cell lung cancer and non-small cell lung cancer), peritoneal cancer, hepatocellular carcinoma, gastric cancer, pancreatic cancer, glioblastoma, cervical cancer, ovarian cancer, liver cancer, bladder cancer, urinary tract cancer, liver tumor, breast cancer, colon cancer, rectal cancer, colorectal cancer, endometrial cancer, uterine cancer, salivary gland cancer, kidney cancer, prostate cancer, vulval cancer, thyroid cancer, anal cancer, penile cancer, melanoma, superficial spreading melanoma, lentigo maligna melanoma, acral melanoma, nodular melanoma, multiple myeloma, B-cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cleaved cell NHL, bulky disease NHL, mantle cell lymphoma, AIDS-related lymphomas, and Waldenstrom's macroglobulinemia), chronic lymphocytic leukemia (CLL), acute lymphoblastic leukemia (ALL), hairy cell leukemia, chronic myeloblastic leukemia, post-transplant lymphoproliferative disorder (PTLD), and abnormal vascular proliferation associated with phakomatoses, edema (such as associated with brain tumors) and Meigs syndrome, brain cancer, head cancer and neck cancer as well as related metastases. In some embodiments, the cancer is breast cancer, peritoneal cancer, fallopian tube cancer, lung cancer, colorectal cancer, biliary tract cancer, bladder cancer, ovarian cancer, prostate cancer or gastric cancer.

The specific dosage and regimen for any particular patient will depend upon a variety of factors including the particular antibody or derivative thereof used, the age and body weight of the patient, general health condition, sex and diet, and the time of administration, frequency of excretion, drug combination and the severity of the particular disease being treated. These factors are judged by medical caregivers included within the scope of those of ordinary skills in the art. The dosage will also depend on the individual patient to be treated, the route of administration, the type of the formulation, the nature of the compound used, the severity of the disease and the efficacy desired. The dosage employed can be determined by pharmacological and pharmacokinetic principles well known in the art.

In some embodiments, a heavy chain of the antibody comprises a sequence set forth in SEQ ID NO: 9, and a light chain of the antibody comprises a sequence set forth in SEQ ID NO: 10. In some embodiments, the anti-HER2 antibody is expressed by a CHO cell with an α-(1,6)-fucosyltransferase gene FUT8 knocked out.

In some embodiments, the method or use comprises: administering to a patient in need thereof a pharmaceutical composition comprising an effective amount of the antibody. In some embodiments, the effective dose is from 50 mg to 1000 mg per dose. In some embodiments, the patient has HER2-positive advanced solid tumors. In some embodiments, the solid tumor is derived from breast cancer or gastric cancer. In some embodiments, the present invention discloses a method for treating HER2-positive advanced solid tumors in a patient in need, which comprises administering an effective amount of the anti-HER2 antibody (or formulation), wherein the effective amount is 50 mg to 1000 mg per treatment cycle. In some embodiments, one treatment cycle is about 1 week, about 2 weeks, about 3 weeks, about 4 weeks, about 5 weeks, about 6 weeks, about 7 weeks, about 8 weeks, or a range between any two of these values (inclusive) or any value therein.

In some embodiments, the anti-HER2 antibody can be formulated into a pharmaceutical composition and administered to a patient in a variety of forms suitable for the chosen route of administration, such as parenteral, intravenous (iv), intramuscular, topical or subcutaneous route. In some embodiments, the anti-HER2 antibody (or formulation) may be intravenously infused. The dose of the anti-HER2 antibody (or formulation) will depend on the properties of the drug, the extent to which the internalization, transport and release of the drug is triggered at the cell surface, and the disease being treated and the conditions of the patient (e.g., age, sex and body weight). In some embodiments, the method for administering the anti-HER2 antibody (or formulation) disclosed herein is intravenous infusion.

In some embodiments, the patient is administered 1-15 mg/kg of the anti-HER2 antibody disclosed herein per treatment cycle. In some embodiments, the dose of the anti-HER2 antibody disclosed herein for each administration is about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, or a range between any two of these values (inclusive) or any value therein. In some embodiments, one treatment cycle is administering once or more times every 1 week to 14 weeks. In some embodiments, the patient is administered 1-15 mg/kg of the anti-HER2 antibody disclosed herein per treatment cycle; wherein one treatment cycle is about 1 week, about 2 weeks, about 3 weeks, about 4 weeks, about 5 weeks, about 6 weeks, about 7 weeks, about 8 weeks, or a range between any two of these values (inclusive) or any value therein. In some embodiments, the patient is administered about 1 mg/kg of the anti-HER2 antibody disclosed herein per treatment cycle; wherein one treatment cycle is administering once every about 2 weeks. In some embodiments, the patient is administered about 3 mg/kg of the anti-HER2 antibody disclosed herein per treatment cycle; wherein one treatment cycle is administering once every about 2 weeks. In some embodiments, the patient is administered about 6 mg/kg of the anti-HER2 antibody disclosed herein per treatment cycle; wherein one treatment cycle is administering once every about 2 weeks. In some embodiments, the patient is administered about 10 mg/kg of the anti-HER2 antibody disclosed herein per treatment cycle; wherein one treatment cycle is administering once every about 2 weeks. In some embodiments, the patient is administered about 15 mg/kg of the anti-HER2 antibody disclosed herein per treatment cycle; wherein one treatment cycle is administering once every about 2 weeks.

In some embodiments, the patient is administered 1-15 mg/kg of the anti-HER2 antibody disclosed herein per treatment cycle; wherein the patient undergoes a plurality of treatment cycles. In some embodiments, the patient is administered 1-15 mg/kg of the anti-HER2 antibody disclosed herein per treatment cycle; wherein the patient undergoes about 2, about 3, about 4, about 5, about 6, or about 7 treatment cycles. In some embodiments, the patient is administered 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg or about 15 mg/kg of the anti-HER2 antibody disclosed herein per treatment cycle, wherein the patient undergoes about 2, about 3, about 4, about 5, or about 6 treatment cycles. In some embodiments, the patient is administered 1 mg/kg of the anti-HER2 antibody disclosed herein per treatment cycle; wherein the patient undergoes about 2, about 3, about 4, about 5, or about 6 treatment cycles. In some embodiments, the patient is administered about 3 mg/kg of the anti-HER2 antibody disclosed herein per treatment cycle; wherein the patient undergoes about 2, about 3, about 4, about 5, or about 6 treatment cycles. In some embodiments, the patient is administered 6 mg/kg of the anti-HER2 antibody disclosed herein per treatment cycle; wherein the patient undergoes about 2, about 3, about 4, about 5, or about 6 treatment cycles. In some embodiments, the patient is administered about 10 mg/kg of the anti-HER2 antibody disclosed herein per treatment cycle; wherein the patient undergoes about 2, about 3, about 4, about 5, or about 6 treatment cycles. In some embodiments, the patient is administered about 15 mg/kg of the anti-HER2 antibody disclosed herein per treatment cycle, wherein the patient undergoes about 2, about 3, about 4, about 5, or about 6 treatment cycles. In some embodiments, the patient is administered once per treatment cycle. In some embodiments, the patient is administered more times, e.g., 2, 3, 4 or 5 times, per treatment cycle. In some embodiments, the patient is administered only 1 or 4 times per treatment cycle.

In some embodiments, the patient receives one treatment cycle of treatment. In some embodiments, the patient receives a plurality of (e.g., 2, 3, or 4) treatment cycles of treatment. In some embodiments, the patient receives treatment until the condition is alleviated and no treatment is required.

In some embodiments, the present invention discloses a method of treating HER2-positive advanced solid tumors, which comprises administering to a patient in need thereof an effective amount of the antibody (or formulation); wherein the effective amount is 50 mg to 1000 mg for a single administration. The dosage schedule and administration mode depend on benefit-risk assessment and general clinical practice guidelines for the antibody (or formulation) in certain patient populations.

In some embodiments, the effective amount per treatment cycle for a patient is 60 mg to 900 mg of the antibody (or formulation). In some embodiments, the effective amount per treatment cycle for a patient is about 60 mg, about 100 mg, about 120 mg, about 180 mg, about 240 mg, about 300 mg, about 360 mg, about 420 mg, about 600 mg, about 700 mg, about 800 mg, about 900 mg, or a range between any two of these values (inclusive) or any value therein. In some embodiments, one treatment cycle is administering once every 1 week to 8 weeks. In some embodiments, the effective amount per treatment cycle is 60 mg to 900 mg; wherein one treatment cycle is about 1 week, about 2 weeks, about 3 weeks, about 5 weeks, about 6 weeks, about 7 weeks, about 8 weeks, or a range between any two of these values (inclusive) or any value therein. In some embodiments, one treatment cycle is 2 weeks. In some treatment regimens, the effective amount per treatment cycle for a patient is 60 mg to 900 mg; wherein one treatment cycle is 2 weeks. In some embodiments, the effective amount per treatment cycle for a patient is about 60 mg, about 100 mg, about 120 mg, about 180 mg, about 240 mg, about 300 mg, about 360 mg, about 420 mg, about 600 mg, about 700 mg, about 800 mg, about 900 mg, or a range between any two of these values (inclusive) or any value therein; wherein one treatment cycle is about 2 weeks.

In some embodiments, the effective amount per treatment cycle for a patient is about 60 mg; wherein one treatment cycle is about 1 week, about 2 weeks, about 3 weeks, or about 4 weeks. In some embodiments, the effective amount per treatment cycle for a patient is 50 mg to 80 mg, such as about 60 mg administered once.

In some embodiments, the effective amount per treatment cycle for a patient is about 180 mg; wherein one treatment cycle is about 1 week, about 2 weeks, about 3 weeks, or about 4 weeks. In some embodiments, the effective amount per treatment cycle for a patient is 150 mg to 200 mg, such as about 180 mg administered once.

In some embodiments, the effective amount per treatment cycle for a patient is about 300 mg; wherein one treatment cycle is about 1 week, about 2 weeks, about 3 weeks, or about 4 weeks. In some embodiments, the effective amount per treatment cycle for a patient is 250 mg to 320 mg, such as about 300 mg administered once.

In some embodiments, the effective amount per treatment cycle for a patient is about 600 mg; wherein one treatment cycle is about 1 week, about 2 weeks, about 3 weeks, or about 4 weeks. In some embodiments, the effective amount per treatment cycle for a patient is 570 mg to 680 mg, such as about 600 mg administered once.

In some embodiments, the effective amount per treatment cycle for a patient is about 900 mg; wherein one treatment cycle is about 1 week, about 2 weeks, about 3 weeks, or about 4 weeks. In some embodiments, the effective amount per treatment cycle for a patient is 880 mg to 940 mg, such as about 900 mg administered once.

In some embodiments, the patient is relieved of symptoms after a single dose is administered. In some embodiments, after a single dose is administered, the patient has not had desired relief of symptoms, then the patient is administered again.

In some embodiments, the initial dose is 600-1000 mg and intravenous infusion is performed for 50-70 min. In some embodiments, the initial dose is about 600 mg, about 630 mg, about 700 mg, about 800 mg, about 840 mg, about 870 mg, about 920 mg, about 980 mg, about 1000 mg, or a range between any two of these values (inclusive) or any value therein. In some embodiments, intravenous infusion is performed for about 50 min, about 53 min, about 56 min, about 60 min, about 63 min, about 67 min, about 70 min, or a range between any two of these values (inclusive) or any value therein.

In some embodiments, the treatment cycle after the first administration of the anti-HER2 antibody disclosed herein is 1-4 weeks, the maintenance dose is 200-500 mg, and the intravenous infusion is performed for 30-60 min. In some embodiments, the treatment cycle after the first administration is about 1 week, about 2 weeks, about 3 weeks, or about 4 weeks. In some embodiments, administration is performed once or twice per treatment cycle. In some embodiments, the maintenance dose is about 200 mg, about 240 mg, about 310 mg, about 380 mg, about 400 mg, about 420 mg, about 460 mg, about 500 mg, or a range between any two of these values (inclusive) or any value therein. In some embodiments, the intravenous infusion is performed for about 30 min, about 33 min, about 42 min, about 49 min, about 54 min, about 60 min, or a range between any two of these values (inclusive) or any value therein.

In some embodiments, the method for treating HER2-positive advanced solid tumors comprises administering to a patient in need thereof an initial dose of 700-900 mg of the anti-HER2 antibody disclosed herein followed by administering about 200-500 mg of the anti-HER2 antibody disclosed herein about every 3 weeks.

### Combination Therapy

In some embodiments, the antibody disclosed herein can be administered in combination with other therapeutic or prophylactic regimens, including administration of one or more antibodies disclosed herein together or in combination with one or more other therapeutic agents or methods.For combination therapy, the antibody may be administered simultaneously or separately with other therapeutic agents. When administered separately, the antibody disclosed herein can be administered before or after administration of another additional therapeutic agent.

In some embodiments, the antibody disclosed herein is administered in combination with trastuzumab. In some embodiments, the initial dose of trastuzumab is 4-10 mg/kg and the intravenous infusion is performed for 80-100 min. In some embodiments, the initial dose of trastuzumab is about 4 mg/kg, about 5 mg/kg, about 6 mg/kg, about 7 mg/kg, about 8 mg/kg, about 9 mg/kg, about 10 mg/kg, or a range between any two of these values (inclusive) or any value therein. In some embodiments, the intravenous infusion is performed for about 80 min, about 81 min, about 84 min, about 87 min, about 90 min, about 92 min, about 95 min, about 97 min, about 98 min, about 100 min, or a range between any two of these values (inclusive) or any value therein. In some embodiments, the treatment cycle after the first administration of trastuzumab is 1-4 weeks, the maintenance dose is 2-8 mg/kg, and the intravenous infusion is performed for 30-90 min. In some embodiments, the treatment cycle after the first administration of trastuzumab is about 1 week, about 2 weeks, about 3 weeks, or about 4 weeks. In some embodiments, administration is performed once or twice per treatment cycle. In some embodiments, the maintenance dose is about 2 mg/kg, about 3 mg/kg, about 4 mg/kg, about 5 mg/kg, about 6 mg/kg, about 7 mg/kg, about 8 mg/kg, or a range between any two of these values (inclusive) or any value therein. In some embodiments, the intravenous infusion is performed for about 30 min, about 40 min, about 50 min, about 60 min, about 70 min, about 80 min, about 90 min, or a range between any two of these values (inclusive) or any value therein.

In some embodiments, the anti-HER2 antibody disclosed herein is administered in combination with trastuzumab-hyaluronidase. In some embodiments, the method for administering trastuzumab-hyaluronidase is subcutaneous administration for 2-5 min. In some embodiments, the subcutaneous administration is performed for about 2 min, about 3 min, about 4 min, about 5 min, or a range between any two of these values (inclusive) or any value therein. In some embodiments, the initial dose of trastuzumab-hyaluronidase is 500 mg/1000 units to 700 mg/1000 units. In some embodiments, the initial dose of trastuzumab-hyaluronidase is about 500 mg/1000 units, about 540 mg/1000 units, about 580 mg/1000 units, about 600 mg/1000 units, about 640 mg/1000 units, about 700 mg/1000 units, or a range between any two of these values (inclusive) or any value therein. In some embodiments, the treatment cycle of trastuzumab-hyaluronidase is 1-4 weeks. In some embodiments, the treatment cycle of trastuzumab-hyaluronidase administration is about 1 week, about 2 weeks, about 3 weeks, or about 4 weeks. In some embodiments, administration is performed once or twice per treatment cycle.

In some embodiments, the anti-HER2 antibody disclosed herein can be used for treating metastatic breast cancer. In some embodiments, the anti-HER2 antibody disclosed herein is used in combination with docetaxel for the treatment of metastatic breast cancer. In some embodiments, the initial dose of docetaxel is 60-70 mg/m² and is administered by intravenous infusion. In some embodiments, the initial dose of docetaxel is about 60 mg/m², about 62 mg/m², about 65 mg/m², about 68 mg/m², about 70 mg/m², or a range between any two of these values (inclusive) or any value therein. In some embodiments, the patient is well-tolerated for the initial dose, the maintenance dose of docetaxel is 80-100 mg/m², and the treatment cycle is 1-4 weeks. In some embodiments, the maintenance dose of docetaxel is about 80 mg/m², about 83 mg/m², about 85 mg/m², about 87 mg/m², about 90 mg/m², about 92 mg/m², about 98 mg/m², about 100 mg/m², or a range between any two of these values (inclusive) or any value therein. In some embodiments, the treatment cycle is about 1 week, about 2 weeks, about 3 weeks, or about 4 weeks.

In other embodiments, the anti-HER2 antibody disclosed herein is administered in combination with a chemotherapeutic agent. In some embodiments, chemotherapeutic agents that can be administered with the antibody disclosed herein include, but are not limited to, antibiotic derivatives (e.g., doxorubicin, bleomycin, daunorubicin and actinomycin D), antiestrogens (e.g., tamoxifen), antimetabolites (e.g., fluorouracil, 5-FU, methotrexate, floxuridine, interferon α-2b, glutamic acid, mithramycin, mercaptopurine and 6-thioguanine), cytotoxic agents (e.g., carmustine, BCNU, lomustine, CCNU, cytarabine, cyclophosphamide, estramustine, hydroxyurea, procarbazine, mitomycin, busulfan, cisplatin and vincristine sulfate), hormones (e.g., medroxyprogesterone, estramustine sodium phosphate, ethinylestradiol, estradiol, megestrol acetate, methyltestosterone, stilbestrol diphosphate, chlorotrianisene and testolactone), nitrogen mustard derivatives (e.g., melphalan, chlorambucil, dichloromethyl diethylamine (mechlorethamine) and thiotepa), steroids and combinations thereof (e.g., betamethasone sodium phosphate), and other compounds (such as dacarbazine, asparaginase, mitotane, vincristine sulfate, vinblastine sulfate and etoposide).

In some embodiments, the antibody disclosed herein is administered in combination with cytokines. Cytokines that can be administered with the antibody disclosed herein include, but are not limited to, IL-2, IL-3, IL-4, IL-5, IL-6, IL-7, IL-10, IL-12, IL-13 and IL-15, and the like.

### Pharmaceutical Composition

The present invention further provides a pharmaceutical composition. Such composition comprises an effective dose of an antibody and a pharmaceutically acceptable carrier. In some embodiments, the pharmaceutical composition further comprises an anti-cancer agent (e.g., an immune checkpoint inhibitor). In some embodiments, the pharmaceutical composition comprises 1%-90% of the antibody of the present invention.

In some embodiments, the term "pharmaceutically acceptable" refers to a substance approved by a government regulatory agency or listed in commonly-recognized pharmacopeias for use in animals, and particularly in humans. In addition, the "pharmaceutically acceptable carrier" generally may be any type of non-toxic solid, semi-solid or liquid filler, diluent, an encapsulating material, or a preparation additive.

The term "carrier" refers to a diluent, adjuvant, excipient or carrier that can be administered to a patient together with the active ingredient. Such pharmaceutical carriers can be sterile liquids, such as water and oils, including oils originated from petroleum, animal, plant or synthesis, such as peanut oil, soybean oil, mineral oil, sesame oil, and the like. Water is a preferred carrier when the pharmaceutical composition is administered intravenously. A saline aqueous solution, a glucose aqueous solution, and a glycerol solution can also be used as a liquid carrier, especially for injection. Suitable pharmaceutical excipients include starch, glucose, lactose, sucrose, gelatin, malt, rice, flour, chalk, silica gel, sodium stearate, glycerol monostearate, talc, sodium chloride, skimmed milk powder, glycerol, propylene, glycol, water, ethanol and the like. If desired, the composition may also comprise a small amount of a wetting agent, an emulsifier, or a pH buffering agent such as acetates, citrates or phosphates. Antibacterials such as benzyl alcohol or methylparaben, antioxidants such as ascorbic acid or sodium bisulfite, chelating agents such as ethylenediamine tetraacetic acid, and tonicity adjusting agents such as sodium chloride or dextrose are also contemplated. Such compositions may be in the form of solutions, suspensions, emulsions, tablets, pills, capsules, pulvises, sustained-release formulations and the like. The composition may be formulated as a suppository using conventional binders and carriers such as triglycerides. Oral formulations may comprise standard carriers such as mannitol, lactose, starch, magnesium stearate, sodium saccharin, cellulose and magnesium carbonate of pharmaceutical grade. Examples of suitable pharmaceutical carriers are described in Remington's Pharmaceutical Sciences by E. W. Martin, which is hereby incorporated herein by reference. Such compositions will contain a clinically effective dose of an antibody or antigen-binding fragment, preferably in purified form, together with an appropriate amount of a carrier, to provide a form suitable for administration to a patient. The formulation should be suitable for the administration mode. The formulation may be enclosed in ampoules, disposable syringes or multiple dose vials made of glass or plastic.

In some embodiments, the composition is formulated into a pharmaceutical composition suitable for intravenous injection into a human body according to conventional steps. A composition for intravenous administration is usually a solution in sterile isotonic aqueous buffer. The composition may also comprise a solubilizer and a local anesthetic such as lidocaine to alleviate the pain at the injection site. In general, the active ingredients are provided in a unit dosage form individually or as a mixture, for example, the active ingredients are encapsulated in sealed containers (such as ampoule bottles or sachets) that can indicate the amount of the active agent, in the form of lyophilized powder or anhydrous concentrate. Where the composition is administered by infusion, the composition can be dispensed in infusion bottles containing sterile, pharmaceutical grade water or saline. Where the composition is administrated by injection, an ampoule bottle containing sterile water or saline for injection can be used, so that the active ingredients can be mixed before administration.

The compound disclosed herein may be formulated in a neutral or salt form. Pharmaceutically acceptable salts include salts formed with anions derived from acids such as hydrochloric acid, phosphoric acid, acetic acid, oxalic acid and tartaric acid, and salts formed with cations derived from, e.g., sodium, potassium, ammonium, calcium or iron hydroxide, isopropylamine, triethylamine, 2-ethylaminoethanol, histidine, and procaine.

### DETAILED DESCRIPTION

The technical solution of the present invention will be further illustrated below through specific examples, which are not intended to limit the protection scope of the present invention. Some nonessential modifications and adjustments made by other people according to the concept of the present invention still fall within the protection scope of the present invention.

The materials, reagents, and the like used in the following examples, unless otherwise stated, can be commercially available, or can be prepared according to known methods.

### Example 1 Expression of HER2 Antigen

An extracellular region protein sequence of HER2 was obtained from NCBI, and a His tag was added for facilitating purification; the sequence of the HER2 antigen is set forth in SEQ ID NO: 11 (see Table 1). The nucleic acid sequence of HER2 antigen was optimized and synthesized according to codon usage bias, the nucleic acid sequence of HER2 antigen was constructed into an expression vector pcDNA3.1 and stably transferred into CHO cells, and high-expression cell strains were screened. Cell supernatant was harvested, and the protein was purified by using a nickel column to obtain the HER2 antigen.

### Example 2 Construction of Vector of Recombinant Humanized Anti-HER2 Antibody

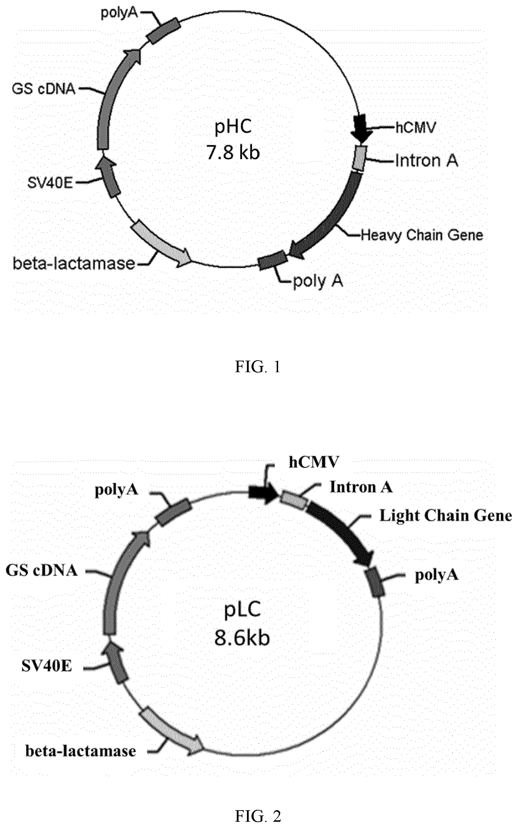

The nucleic acid sequences of the antibody light chain and the antibody heavy chain were optimized and synthesized according to the codon usage bias. With reference to method in Wood et al., High level synthesis of immunoglobulins in Chinese hamster ovary cells, J Immunol. 145(9):3011(1990) and the like, the nucleic acid sequences of the antibody light chain and the antibody heavy chain were constructed into expression vectors, respectively, the plasmid containing the nucleic acid sequence of the heavy chain was pHC (shown in FIG. 1), and the plasmid containing the nucleic acid sequence of the light chain was pLC (shown in FIG. 2); wherein the heavy chain sequence of the anti-HER2 antibody was set forth in SEQ ID NO: 9, and the light chain sequence of the anti-HER2 antibody was set forth in SEQ ID NO: 10. The two plasmids pHC and pLC were combined into a complete plasmid by enzyme digestion by adopting a conventional molecular biology method to obtain the plasmid pHL. The amino acid sequences of the present invention are shown in Table 1, and the corresponding nucleic acid sequences are shown in Table 2.

**Table 1 Amino acid sequence of antigen or antibody**

| Name | No. | Amino acid sequence |
|---|---|---|
| HCDR1 | SEQ ID NO: 1 | GFTFTDYT |
| HCDR2 | SEQ ID NO:2 | VNPNSGGSIYNQRFK |
| HCDR3 | SEQ ID NO:3 | LGPSFYFDY |
| LCDR1 | SEQ ID NO:4 | KASQDVSIGVA |
| LCDR2 | SEQ ID NO:5 | ASYRYTG |
| LCDR3 | SEQ ID NO:6 | QQYYIYPYTF |
| VH | SEQ ID NO:7 | |
| VL | SEQ ID NO:8 | DIQMTQSPSSLSASVGDRVTITCKASQDVSIGVAW YQQKPGKAPKLLIYSASYRYTGVPSRFSGSGSGTD FTLTISSLQPEDFATYYCQQYYIYPYTFGQGTKVEI KR |
| Heavy chain full length | SEQ ID NO:9 | EVQLVESGGGLVQPGGSLRLSCAASGFTFTDYTM DWVRQAPGKGLEWVADVNPNSGGSIYNQRFKGR FTLSVDRSKNTLYLQMNSLRAEDTAVYYCARNLG PSFYFDYWGQGTLVTVSSASTKGPSVFPLAPSSKST SGGTAALGCLVKDYFPEPVTVSWNSGALTSGVHT FPAVLQSSGLYSLSSVVTVPSSSLGTQTYICNVNHK PSNTKVDKKVEPKSCDKTHTCPPCPAPELLGGPSV FLFPPKPKDTLMISRTPEVTCVVVDVSHEDPEVKFN WYVDGVEVHNAKTKPREEQYNSTYRVVSVLTVL HQDWLNGKEYKCKVSNKALPAPIEKTISKAKGQP REPQVYTLPPSREEMTKNQVSLTCLVKGFYPSDIA VEWESNGQPENNYKTTPPVLDSDGSFFLYSKLTVD KSRWQQGNVFSCSVMHEALHNHYTQKSLSLSPGK |
| Light chain full length | SEQ ID NO:10 | DIQMTQSPSSLSASVGDRVTITCKASQDVSIGVAW YQQKPGKAPKLLIYSASYRYTGVPSRFSGSGSGTD FTLTISSLQPEDFATYYCQQYYIYPYTFGQGTKVEI KRTVAAPSVFIFPPSDEQLKSGTASVVCLLNNFYPR EAKVQWKVDNALQSGNSQESVTEQDSKDSTYSLS STLTLSKADYEKHKVYACEVTHQGLSSPVTKSFNR GEC |
| HER2 antigen | SEQ ID NO:11 | TQVCTGTDMKLRLPASPETHLDMLRHLYQGCQVV QGNLELTYLPTNASLSFLQDIQEVQGYVLIAHNQV RQVPLQRLRIVRGTQLFEDNYALAVLDNGDPLNN TTPVTGASPGGLRELQLRSLTEILKGGVLIQRNPQL |
| | | CYQDTILWKDIFHKNNQLALTLIDTNRSRACHPCS PMCKGSRCWGESSEDCQSLTRTVCAGGCARCKGP LPTDCCHEQCAAGCTGPKHSDCLACLHFNHSGICE LHCPALVTYNTDTFESMPNPEGRYTFGASCVTACP YNYLSTDVGSCTLVCPLHNQEVTAEDGTQRCEKC SKPCARVCYGLGMEHLREVRAVTSANIQEFAGCK KIFGSLAFLPESFDGDPASNTAPLQPEQLQVFETLE EITGYLYISAWPDSLPDLSVFQNLQVIRGRILHNGA YSLTLQGLGISWLGLRSLRELGSGLALIHHNTHLCF VHTVPWDQLFRNPHQALLHTANRPEDECVGEGLA CHQLCARGHCWGPGPTQCVNCSQFLRGQECVEEC RVLQGLPREYVNARHCLPCHPECQPQNGSVTCFGP EADQCVACAHYKDPPFCVARCPSGVKPDLSYMPI WKFPDEEGACQPCPINCTHSCVDLDDKGCPAEQR ASPLTHHHHHH |

**Table 2 Nucleic acid sequences of the antibodies**

| Name | No. | Nucleic acid sequence |
|---|---|---|
| Light chain of anti-HER2 antibody | SEQ ID NO:12 | gatatccagatgacccagagcccttcctccctgagcgctagcgtgggcgaccg ggtgaccatcacctgcaaggctagccaggacgtgtccatcggcgtggcctggt accagcagaagcccggcaaggcccccaagctgctgatctactccgcttcctatc ggtataccggcgtgccttccaggttctccggcagcggctccggcaccgacttca ccctgaccatctcctccctgcagcctgaggacttcgccacctactactgtcagca gtactacatctacccttacaccttcggccagggcaccaaggtggagatcaaacg tacggtggctgcaccatctgtcttcatcttcccgccatctgatgagcagttgaaat ctggaactgcctctgttgtgtgcctgctgaataacttctatcccagagaggccaaa gtacagtggaaggtggataacgccctccaatcgggtaactcccaggagagtgt cacagagcaggacagcaaggacagcacctacagcctcagcagcaccctgac gctgagcaaagcagactacgagaaacacaaagtctacgcctgcgaagtcacc catcagggcctgagctcgcccgtcacaaagagcttcaacaggggagagtgt |
| Heavy chain of anti-HER2 antibody | SEQ ID NO:13 | gaggtgcagctggtggagtccggcggactggtgcagccaggaggaagc ctgaggctgtcctgcgccgctagcggcttcaccttcaccgattacaccatggact gggtgaggcaggcccctggcaagggcctggagtgggtggctgacgtgaatcc caatagcggcggctccatctacaatcagaggttcaagggcaggtttaccctgtc cgtggataggagcaagaataccctgtacctgcagatgaactccctgagggccg aggacaccgccgtgtattattgtgctcggaatctgggccctagcttttatttcgatt attggggccagggtaccctggtcaccgtgtctagtgctagtactaaggggccttc agtgtttccactggcaccctcatccaaatccaccagcggcggaacagcagccct gggttgtctggtgaaggactacttcccagaacccgtcaccgtgtcatggaactcc ggcgcactgacatctggagtccacacttttcctgccgtgctgcagagctctggg ctgtactccctgagttcagtggtcactgtcccatccagctctctgggtacacagac ttatatctgcaacgtgaatcacaagccaagtaataccaaagtcgacaagaaagt ggagcccaagtcatgtgataaaacccatacatgccccccttgtcctgcaccaga actgctgggaggtccaagcgtgttcctgtttccacccaagcctaaagacacact |
| | | gatgattagtaggactcccgaggtcacctgcgtggtcgtggacgtgtcccacga ggatcctgaagtcaagtttaactggtacgtggatggcgtcgaagtgcataatgct aagaccaaaccaagagaggaacagtacaactccacttatcgcgtcgtgagcgt cctgaccgtgctgcatcaggattggctgaacggcaaggagtataagtgcaaagt gagcaataaggctctgcccgcacctatcgagaaaaccatttctaaggcaaaag gacagcctcgagaaccacaggtgtacacactgcctccaagccgtgaggaaat gaccaagaaccaggtctctctgacatgtctggtgaaaggcttctatccttcagac attgctgtggagtgggaatccaatggacagccagagaacaattacaagaccac accccctgtgctggacagcgatggctctttctttctgtattctaagctgaccgtgga taaaagtcggtggcagcagggaaatgtcttttcttgtagtgtgatgcacgaagca ctgcacaaccactacacccagaaatcactgtcactgtctccagggaag |

### Example 3 Construction of Stable Cell lines

Plasmid pHL was subjected to an enzyme digestion to obtain a linearized plasmid. A CHO cell (such as CHO-k1 cell) and the CHO cell with an α-(1,6)-fucosyltransferase gene FUT8 knocked out were prepared, and the linearized pHL plasmid was transfected into the two cells by electroporation. After the electrotransfection, the two cells were resuspended in CHO-AGT (Gibco, Cat.# 12490025) medium containing 1× GS (Specialty Media, Cat.# GSS1016-C) and distributed evenly in 40 96-well plates at a density of 2500 cells per well, and the volume of each well was 100 µL. The cell culture plates were placed in a 37 °C incubator with 8% CO₂. After 48 h, the culture was continued by replenishing 100 µL of CHO-AGT medium (1× GS and MSX (methionine sulfoximine) at a final concentration of 50 µM). Cells in the 96-well cell culture plate formed visible cell clone clusters after about 16-20 days. A plurality of high-expression cell strains were screened out using ELISA, and subjected to staged amplification culture to obtain the target protein. The antibody expressed by the CHO cell was named as BAT0303, and the antibody expressed by the CHO cell in which an α-(1,6)-fucosyltransferase gene FUT8 was knocked out was named as BAT0303F. Among them, a method for knocking out the α-(1,6)-fucosyltransferase gene FUT8 of CHO is referred to Patent WO2019029713A. Cell culture supernatant was subjected to Protein A chromatography, anion chromatography, cation chromatography and other steps to obtain the purified antibody.

### Example 4 Determination of Molecular Weight of Antibodies

The concentration of antibodies was diluted to 1 mg/mL and effectively separated using a C₄ chromatographic column and qualitatively detected using LC-MS. The mobile phase was as follows: phase A was 0.1% FA (formic acid)/ultrapure water, and phase B was 0.1% FA/acetonitrile; the chromatographic column was Waters ACQUITY C₄ (2.1 × 100 mm, 1.7 µm), the column temperature was 70 °C, the flow rate was 0.3 mL/min, and the injection volume was 5 µL (i.e., 5 µg). Data acquired by UNIFI was processed using UNIFI.

As shown in Table 3, the difference between the molecular weight of Antibody BAT0303F and the theoretical value was about 2 Da, which fell within the normal error range and met the requirements.

**Table 3 Results of Complete Molecular Weight of BAT0303F**

| Antibody sample | Theoretical molecular weight | Measured molecular weight | Error (Da) |
|---|---|---|---|
| BAT0303F-1 | 147793.82 | 147795.87 | 2.05 |
| BAT0303F-2 | | 147795.78 | 1.96 |
| BAT0303F-3 | | 147796.05 | 2.23 |

### Example 5 Glycosylation Modification Detection

Separation was performed using HILIC-HPLC chromatography, and the glycoform of the expression protein of a plurality of cell strains and the glycoform of a reference product (commercially available Perjeta^{®}) were detected by fluorescence detector.

Table 4 only lists the partial glycoform percentage content of a group of antibodies BAT0303F; wherein the content of G0 glycoform was no less than 60%, the content of GO-GN glycoform was 4%-7%, the content of G1' glycoform was 6%-12%, the content of G1 glycoform was 8%-12%, and the content of G2 glycoform was no more than 3%.

**Table 4 Partial glycoform percentage content**

| Antibody sample | G0F-GN | GO-GN | G0 | G0F | Man5 | G1 | G1' | G1F | G1F' | G2 | G2F |
|---|---|---|---|---|---|---|---|---|---|---|---|
| Perjeta^{®} | 3.28 | 0.40 | 3.26 | 77.58 | 1.1 | 0.54 | 0.14 | 7.34 | 3.73 | ND | 0.77 |
| BAT0303F | ND | 5.35 | 72.56 | ND | 2.27 | 8.94 | 8.94 | ND | ND | 0.76 | ND |

| | | | | | | | | | | | |
|---|---|---|---|---|---|---|---|---|---|---|---|
| Note: ND: not detectable. | | | | | | | | | | | |

### Example 6 Determination of Acid Peaks and Basic Peaks

1) Ion exchange chromatography analysis was performed at 280 nm using a ProPacTM WCX-10 chromatography column (4 × 250 mm, 10 µm) (mobile phase A: 20 mmol/L sodium phosphate buffer, pH 7.0, mobile phase B: 20 mmol/L sodium phosphate buffer, 100 mmol/L NaCl, pH 7.0, flow rate: 0.8 mL/min, retention time: 5-35 min, and 10% -50% of mobile phase B). Two batches of BAT0303F and two batches of Perjeta^{®} were selected for detection, and the detection results of the BAT0303F and Perjeta^{®} are shown in Table 5.

As shown in Table 5, BAT0303F differed from Perjeta^{®} in acid and basic peak distribution, and the basic peak of BAT0303F was significantly lower than Peijeta^{®}, indicating that both protein modifications had certain differences.

**Table 5 Peak percentage content**

| Name | Sample batch | Acidic variant% | Main peak% | Basic variant% |
|---|---|---|---|---|
| BAT0303F | Batch 1 | 18.47 | 78.83 | 2.70 |
| | Batch 2 | 16.88 | 78.54 | 4.58 |
| Perjeta^{®} | H0338B02 | 19.79 | 68.03 | 12.18 |
| | H0323B07 | 19.18 | 68.84 | 11.99 |

| | | | | |
|---|---|---|---|---|
| Note: the data in the table is integrated by a computer with two decimal places reserved. | | | | |

2) CBP enzyme digestion identification: a proper amount of test sample was taken and diluted with mobile phase A (20 mmol/L sodium phosphate buffer); the mixture was added with 5 mg/mL of CBP (recombinant carboxypeptidase B, RCPB 01, available from Shanghai Yaxin Biotechnology Co., Ltd.), enabling the mass ratio of the test sample to the CBP to be 50:1 and the final concentration of the antibody to be 1 mg/mL. The mixture was uniformly mixed, incubated in a 37 °C water bath for 30 min, and analyzed using the above ion exchange chromatography. The detection results of BAT0303F and Peijeta^{®} are shown in Table 6 (the content reduction of basic peaks was relative to basic peaks in Table 5).

As shown in Table 6, the results indicated that there was little difference in the basic lysine content at terminus between BAT0303F and Perjeta^{®}, and that basic peaks may be caused by other modifications.

**Table 6 Peak percentage content**

| Name | Sample batch | Acidic variant% | Main peak% | Basic variant% | Basic peak reduction% |
|---|---|---|---|---|---|
| BAT0303F | Batch 1+ CPB | 18.14 | 80.28 | 1.57 | 1.13 |
| | Batch 2+ CPB | 16.88 | 79.92 | 3.20 | 1.38 |
| Perjeta^{®} | H0338B02+CPB | 20.35 | 70.34 | 9.20 | 2.98 |
| | H0323B07+CPB | 19.76 | 71.29 | 8.96 | 3.03 |

| | | | | | |
|---|---|---|---|---|---|
| Note: the data in the table is integrated by a computer with two decimal places reserved. | | | | | |

### Example 6 Binding Activity of Antibodies

0.75 µg/mL Her2 antigen coating solution was prepared using PBS, and each well in the plate was paved with 100 µL of solution and then coated overnight at 4 °C. The plate was subsequently blocked with 3% BSA at 37 °C for 2 h, and the BAT0303F and BAT0303 antibodies at the initial concentration of 2 µg/mL were diluted in gradients for 9 gradients. HRP-labeled anti-Fc secondary antibody (sigma, Cat.# A7164-1ML) was added in a ratio of 1: 10000, and the mixture was incubated at 37 °C for 1 h. The supernatant was removed. The precipitate was washed with PBST (a solution of 0.05% Tween-20 in PBS) several times, added with TMB developing solution, incubated at 37 °C for 5-10 min, and then added with 2M H₂SO₄ to terminate the development.The plate was tested by a microplate reader, simultaneously the absorbance value of each well was read, and the wavelength of detection was 450 nm.

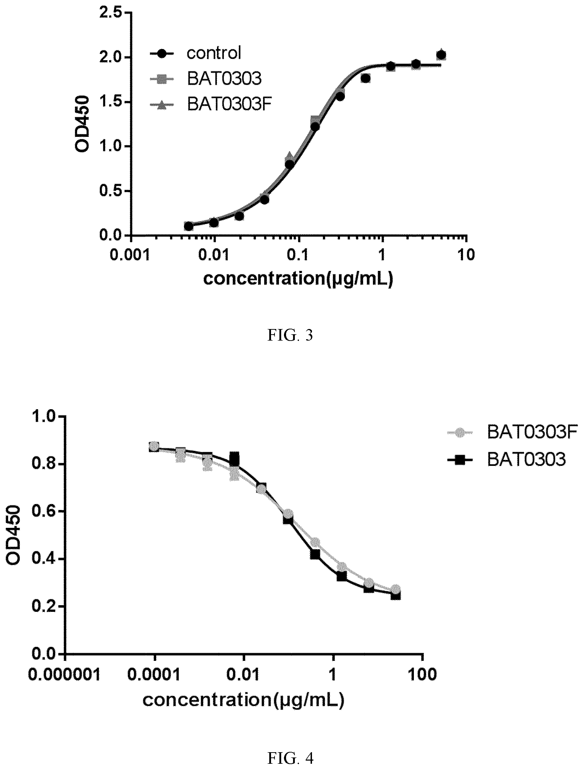

As shown in FIG. 3, Antibody BAT0303F had similar binding capacity to Her2 antigen as compared to Peijeta^{®} and antibody BAT0303; defucosylation did not affect the binding activity of Antibody BAT0303F.

### Example 7 Assay of Affinity

In vitro binding activity of Antibody BAT0303F to HER2 antigen was tested using BIAcore (GE Healthcare, T200). The concentration of the test antibodies was diluted to 5 µg/mL by HBS-EP (0.01 M HEPES, 0.15 M NaCl, 3 mM EDTA, 0.005% P20, pH 7.4), and the concentration of the HER2-His antigen was diluted to 100 nM by HBS-EP, which was taken as an initial concentration for 2-fold gradient dilution to obtain HER2-His diluents at concentrations of 100 nM, 50 nM, 25 nM, 12.5 nM, 6.25 nM and 3.125 nM. Detection was preformed using a Protein A (GE Healthcare, Cat.# 29127555) chip, and 5 µg/mL of antibody diluents was passed through the experimental flow paths (Fc2 and Fc4) at a flow rate of 10 µL/min for 15 s so that the capture amount was about 600 RU; then the flow rate was adjusted to 30 µL/min, antigen diluents with different concentrations (0 nM, 3.125 nM, 6.25 nM, 12.5 nM, 25 nM, 50 nM and 100 nM) were sequentially added and simultaneously passed through the surfaces of experimental flow paths (Fc2 and Fc4) and reference flow paths (Fc1 and Fc3) with an association time of 180 s and a dissociation time of 600 s, and finally the chip was subjected to regeneration by the Glycine 1.5 buffer for 60 s and entered the next cycle. Experiment results were analyzed using data analysis software Evaluation software 3.1, the sensing signals acquired by the sample experiment flow path passed through reference flow paths, blank double subtraction was performed on samples, and fitting was preformed using a kinetic "1:1" model to obtain the kinetic parameters of the affinity association of each antibody sample with the HER2.

As shown in Table 7, Perjeta^{®} and Antibody BAT0303F were of similar affinity to Her2 antigen.

**Table 7 Kinetic parameters of affinity binding**

| Samples | kₐ (1/Ms) | k_{d} (1/s) | K_{D} (M) |
|---|---|---|---|
| Perjeta^{®} | 5.34e+04 | 2.30e-04 | 4.30e-09 |
| BAT0303F | 4.58e+04 | 1.48e-04 | 3.24e-09 |

| | | | |
|---|---|---|---|
| Note: kₐ: association rate; k_{d}: dissociation rate; K_{D}: association-dissociation equilibrium constant, i.e., affinity. | | | |

### Example 8 Inhibition of Cell Proliferation

MDA-MB-175VII cells (from National Infrastructure of Cell Line Resource) were cultured in advance, and when the cells were paved to 60%-80% of plate, the cells were digested, the digestion was terminated with medium containing 10% FBS, the cell density was adjusted to 6 × 10⁴ cells/mL using medium containing 2% FBS, and the cells were paved to 96 cell culture plates at a density of 6000 cells/well. After the plate was cultured in the incubator for 12-24 h, the test antibody sample was added and diluted in gradient, after the plate was cultured for three days, the supernatant was discarded, the remaining was added into a culture medium containing 10% CCK8 (DOJINDO, CK04), then subjected to color development in the incubator for 2-4 h, and OD450 values were read using a microplate reader.

As shown in FIG. 4, antibodies BAT0303F and BAT0303 can specifically bind to HER2 extracellular domain II, thereby inhibiting the proliferation of MDA-MB-175VII cells.

### Example 9 Assay of In Vitro ADCC Effect

HCC1954 cells (Nanjing Cobioer) and NCI-N87 cells (from Cell Bank of Type Culture Collection Committee of the Chinese Academy of Sciences) were cell lines with high expression of HER2 antigen, and when the growth state of the cells was better, the cells were digested and plated at 6000 cells/well, and were co-cultured with target cells (HCC1954 cells and NCI-N87 cells) at a density of 30000 cells/well according to PBMC (peripheral blood mononuclear cells) for 3-4 h, then antibodies BAT0303 and BAT0303F, and trastuzumab analog BAT-Her (sequence of BAT-Her antibody was the same as that of trastuzumab, BAT-Her was expressed by CHO cells) were diluted in gradient, the cells were cultured in an incubator at 37 °C for 3 days, the supernatant was removed, a culture medium containing 10% CCK8 was added and the cell was incubated for 2-4 h, and the OD450 values were read by an enzyme reader.

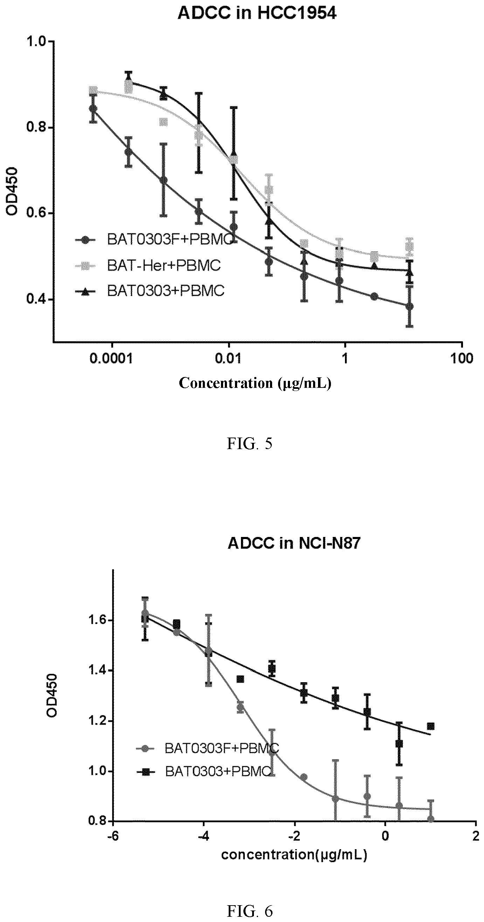

As shown in FIGs. 5 and 6, antibody BAT0303 had a similar ADCC effect as BAT-Her, but Antibody BAT0303F killed target cells much more strongly than antibodies BAT0303 and BAT-Her.

### Example 10 Assay of Synergistic Efficacy with BAT-Her

BAT0303F and BAT-Her targeted domain II and domain IV of HER2, respectively, which inhibit cell proliferation by different mechanisms of action, and it was investigated in this Example whether BAT0303F has a synergistic effect with BAT-Her. In NCI-N87 cells, plating was performed in advance, and antibody gradient diluents of BAT0303F, BAT-Her and BAT0303F+BAT-Her were added. After 3 days of culture in an incubator, supernatant was removed, the plate was added with a culture medium containing 10% CCK8 and subjected to color development for 3-5 h, and the OD450 values were read by an enzyme reader.

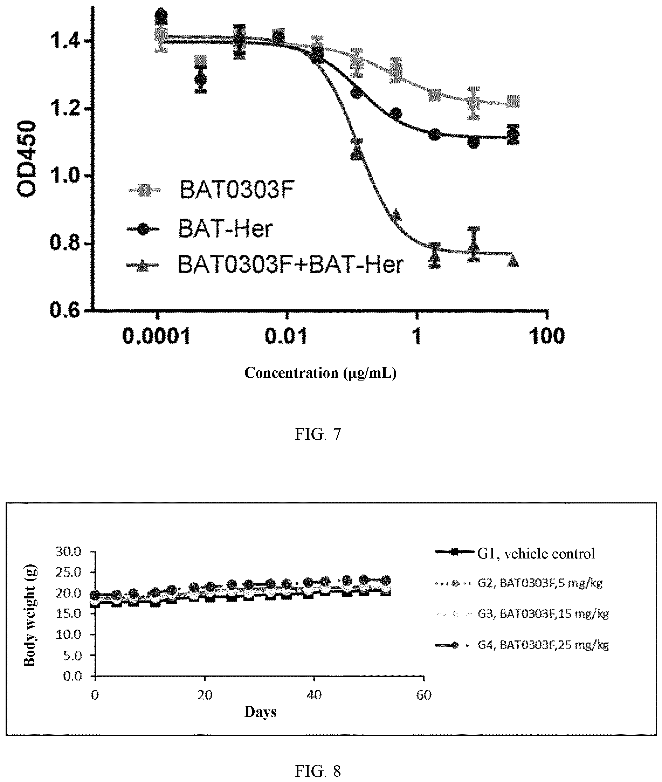

As shown in FIG. 7, both antibodies BAT0303F and BAT-Her inhibited the growth of NCI-N87, and both antibodies BAT0303F and BAT-Her significantly inhibited the growth of NCI-N87 when administered in combination.

### Example 11 Assay of Receptor Affinity

In order to assay the affinity of Antibody BAT0303F to Fc receptor and complement C1q, differences and similarities in affinity of Antibody BAT0303F and Perjeta^{®} to neonatal Fc receptor protein Rn (FcRn, Cat.# FCM-H82W4 from ACROBiosystems), Fc receptor protein gamma IIIa (Fc gamma RIIIa, Cat.# CDA-H82E9 from ACROBiosystems), Fc gamma RIIa (Cat.# CDA-H82E6 from ACROBiosystems) and complement C1q (Cat.# A-400 from quidel) were tested and compared using BLI (Bio-Layer Interferometry). After data were collected, data analysis software Acquisition 8.2 of an instrument was used for analyzing the data, signals collected by Baseline were used as baselines, reference signals were deducted, and group analysis and fitting were performed on the obtained data.

As shown in Table 8, compared with Peijeta^{®}, the affinity of the Antibody BAT0303F for the receptor FcγRIIIa(V158) was significantly improved, but the affinity thereof to several other receptors was not significantly different; after fucose in the antibody was knocked out, the affinity of the antibody for FcγRIIIa(V158) was specifically improved.

**Table 8 Result of affinity of BAT0303F for Fc receptor and C 1q**

| Receptor | Affinity K_{D} (M) | |
|---|---|---|
| | BAT0303F | Perjeta^{®} |
| FcRn | 3.03E-07 | 2.56E-07 |
| FcγRIIIa(V158) | 3.68E-08 | 1.18E-07 |
| FcγRIIIa(H131) | 9.50E-07 | 8.70E-07 |
| C1q | 7.57E-09 | 6.38E-09 |

### Example 12 Efficacy of Xenograft Tumor

Calu-3 cells (human non-small cell lung cancer cells) were cultured in MEM medium (supplemented with 0.01 mM NEAA) containing 10% fetal bovine serum, and Calu-3 cells in exponential growth phase were collected and PBS was resuspended to a suitable concentration for subcutaneous tumor inoculation on BALB/c nude mice (Beijing AniKeeper Biotech Co., Ltd.).

The test mice were inoculated subcutaneously at the right anterior scapula on the right dorsum with 1 × 10⁷ Calu-3 cells, which were resuspended in PBS 1:1 mixed with matrigel (0.2 mL/mouse). The growth of the tumors was observed periodically. When the mean volume of the tumors reached 185 mm³, the mice were randomly grouped for administration according to the tumor size and the mouse weight. The day of grouping was defined as Day 0 and the administration was started at Day 0. The mice were administered once a week for a total of 4 doses, administration was stopped on day 21, and tumor inhibition effect was continuously observed until D35; the results of tumor inhibition are shown in Table 9 and FIG. 8.

**Table 9**

| Experimental group | Mean tumor volume^{a} (Day 0) | Mean tumor volume (Day 28)^{b} | Mean tumor volume (Day 53) | TGI (%)^{c} | *P* Value (compared to control group)^{d} |
|---|---|---|---|---|---|
| G1 Vehicle control group | 184.61±5.41 | 860.85±110.24 | 2241.92±214.31 | - | - |
| G2 BAT0303F, 5mg/kg | 184.63±3.42 | 154.87±39.13 | 485.69±153.59 | 85.37 | 7.62e-03** |
| G3 BAT0303F, 15mg/kg | 184.67±3.53 | 73.76±11.94 | 161.81±47.62 | 101.11 | 2.18e-05*** |
| G4 BAT0303F, 25mg/kg | 184.61±3.65 | 103.53±22.17 | 379.64±136.02 | 90.52 | 6.44e-04*** |

| | | | | | |
|---|---|---|---|---|---|
| Note: a. data are represented by "mean ± standard error"; b. mean tumor volume after the stop of administration cycle (Day 28); c. TGI% = [1 - (Ti - T0)/(Ci - C0)] × 100, wherein T0 and C0 are the mean tumor volumes at Day 0 of the administration group and vehicle control group, respectively, and Ti and Ci are the mean tumor volumes at Day 53 of the administration group and vehicle control group, respectively; d. *P<0.05, **P<0.01, ***P<0.001, ^{ns}*P*>0.05 (compared to tumor volume in vehicle control group (day 53)). | | | | | |

As shown in Table 9, the group administered with Antibody BAT0303F significantly inhibited the growth of tumor cells, and Antibody BAT0303F administered at 5 mg/kg inhibited the growth of xenograft tumor to the maximum extent, reaching the saturation dose; as shown in FIG. 8, tumor-bearing mice tolerated well at all doses tested for the Antibody BAT0303F.

### Example 13 Clinical Study

This study is an open-label and dose escalation phase I clinical trial for studying the safety, tolerability, and pharmacokinetic characteristics of BAT0303F antibody for patients with locally advanced/metastatic HER2-positive solid tumors.

The administration mode adopted in this study was: intravenous infusion, administered on the first day of each cycle, 14 days per cycle. The dose was sequentially set to be 1 mg/kg, 3 mg/kg, 6 mg/kg, 10 mg/kg and 15 mg/kg, the 1 mg/kg and 3 mg/kg dose groups adopted a rapid escalating setting, and the 6 mg/kg dose group follows a "3+3" escalating setting at first.

The evaluation indexes of the anti-tumor efficacy comprise:
Best overall response (BOR): complete remission (CR), partial remission (PR), disease stabilization (SD), and disease progression (PD);
Objective response rate (ORR): a proportion of subjects in complete response (CR) and partial response (PR);
Duration of response (DOR); and
Treatment past progression: progression-free survival (PFS) during administration.

### Inclusion criteria

Subjects need to meet all of the following criteria for inclusion:
1) Patients with advanced solid tumors (preferably breast cancer, gastric cancer) that are histopathologically and/or cytologically confirmed to be positive for HER2. HER2-positive (HER2-positive is defined as FISH+, or IHC3+ or IHC2+, and results of gene amplification detection are positive);
2) Tumors with at least 1 measurable lesion according to RECIST (response evaluation criteria in solid tumor) 1.1 edition;
3) Eastern Cooperative Oncology Group (ECOG) score for the physical strength is 0-1 point; and
4) Patients with sufficient bone marrow, liver, kidney and blood coagulation functions, which are defined as follows:

| **System** | **Laboratory parameter value** |
|---|---|
| **Blood system** (no blood transfusion, no hematopoietic stimulating factor, and no medication to correct blood cell counts within 14 days before blood draw) | |
| Absolute value of neutrophil (NEU) count | ≥1.5x10⁹/L |
| Platelet (PLT) count | ≥ 100 x10⁹/L |
| Hemoglobin (HB) | ≥ 90g/L |

| **Liver** | |
|---|---|
| Total bilirubin (TBIL) | ≤ 1.5 ULN |
| Without liver metastasis | |
| Aspartate transaminase (AST) | ≤2.5 ULN |
| Alanine transaminase (ALT) | ≤2.5 ULN |
| Liver metastasis | |
| Aspartate transaminase (AST) | ≤5.0 ULN |
| Alanine transaminase (ALT) | ≤5.0 ULN |
| **Kidney** | |
| Creatinine (Cr) | ≤1.5ULN |
| Blood urea nitrogen (BUN) | ≤1.5ULN |

| **Blood coagulation** | |
|---|---|
| International normalized ratio (INR) | ≤1.5ULN |
| Activated partial thromboplastin time (APTT) | ≤1.5ULN |

5) Left ventricular ejection fraction (LVEF) of the echocardiography is ≥50%.

### Exit criteria

Subjects were considered to be withdrawn from the study if they had any of the following events during treatment:
1) any AE that prevents the subject from continuing to participate in the study;
2) any reason that causes the subject's study treatment to be delayed for more than 21 days;
3) concurrent disease or changes in the subject's condition, which causes the investigator believes that the subject is no longer eligible for study treatment;
4) lactation or pregnancy;
5) poor subject compliance with study procedures and study treatments;
6) the subject or researcher requests discontinuation of study treatment;
7) the subject may, for any reason, require other anti-tumor drugs or measures;
8) disease progression requires other anti-tumor treatments; and
9) other reasons.

## Claims

1. An antibody, specifically binding to HER2, and comprising: (a) an HCDR1 comprising a sequence set forth in SEQ ID NO: 1, (b) an HCDR2 comprising a sequence set forth in SEQ ID NO: 2, (c) an HCDR3 comprising a sequence set forth in SEQ ID NO: 3, (d) an LCDR1 comprising a sequence set forth in SEQ ID NO: 4, (e) an LCDR2 comprising a sequence set forth in SEQ ID NO: 5, and (f) an LCDR3 comprising a sequence set forth in SEQ ID NO: 6;
wherein the antibody has a fucosylation level of 0%-5%.

2. The antibody according to claim 1, wherein the antibody has a fucosylation level of 0%.

3. An antibody, specifically binding to HER2, and comprising: (a) an HCDR1 comprising a sequence set forth in SEQ ID NO: 1, (b) an HCDR2 comprising a sequence set forth in SEQ ID NO: 2, (c) an HCDR3 comprising a sequence set forth in SEQ ID NO: 3, (d) an LCDR1 comprising a sequence set forth in SEQ ID NO: 4, (e) an LCDR2 comprising a sequence set forth in SEQ ID NO: 5, and (f) an LCDR3 comprising a sequence set forth in SEQ ID NO: 6;
wherein the antibody comprises no less than 60% G0.

4. The antibody according to claim 3, wherein the content of G0 glycoform in the antibody is 61%-75%.

5. The antibody according to claim 3, wherein the content of G0 glycoform in the antibody is 72%-73%.

6. The antibody according to any one of claims 1-5, wherein the content of G0-GN glycoform is 3%-7%, the content of G1 glycoform is 6%-12%, the content of G1' glycoform is 6%-12%, and the content of G2 glycoform is no more than 3%.

7. The antibody according to claim 6, wherein the content of G0-GN glycoform is 5%-6%, the content of G1 glycoform is 8%-9%, the content of G1' glycoform is 8%-9%, and the content of G2 glycoform is 0-1%.

8. The antibody according to any one of claims 1-7, wherein a heavy chain variable region of the antibody comprises a sequence set forth in SEQ ID NO: 7, or a sequence having at least 80% identity to the sequence set forth in SEQ ID NO: 7, or a sequence having one or more conservative amino acid substitutions as compared to the sequence set forth in SEQ ID NO: 7; and/or
a light chain variable region of the antibody comprises a sequence set forth in SEQ ID NO: 8, or a sequence having at least 80% identity to the sequence set forth in SEQ ID NO: 8, or a sequence having one or more conservative amino acid substitutions as compared to the sequence set forth in SEQ ID NO: 8.

9. The antibody according to claim 8, wherein a heavy chain of the antibody comprises a sequence set forth in SEQ ID NO: 9, or a sequence having at least 80% identity to the sequence set forth in SEQ ID NO: 9, or a sequence having one or more conservative amino acid substitutions as compared to the sequence set forth in SEQ ID NO: 9; and/or
a light chain of the antibody comprises a sequence set forth in SEQ ID NO: 10, or a sequence having at least 80% identity to the sequence set forth in SEQ ID NO: 10, or a sequence having one or more conservative amino acid substitutions as compared to the sequence set forth in SEQ ID NO: 10.

10. The antibody according to any one of claims 1-9, wherein the antibody is expressed by a CHO cell with α-(1,6)-fucosyltransferase gene FUT8 knocked out.

11. An antibody composition, wherein an antibody in the antibody composition specifically binds to HER2, and comprises: (a) an HCDR1 comprising a sequence set forth in SEQ ID NO: 1, (b) an HCDR2 comprising a sequence set forth in SEQ ID NO: 2, (c) an HCDR3 comprising a sequence set forth in SEQ ID NO: 3, (d) an LCDR1 comprising a sequence set forth in SEQ ID NO: 4, (e) an LCDR2 comprising a sequence set forth in SEQ ID NO: 5, and (f) an LCDR3 comprising a sequence set forth in SEQ ID NO: 6;
the content of main peak in the antibody composition is at least 72%.

12. The antibody composition according to claim 11, wherein the content of main peak in the antibody composition is 73%-82%.

13. The antibody composition according to claim 11 or 12, wherein the content of the basic variants in the antibody composition is no more than 6%.

14. The antibody composition according to any one of claims 11-13, wherein a heavy chain variable region of the antibody comprises a sequence set forth in SEQ ID NO: 7, or a sequence having at least 80% identity to the sequence set forth in SEQ ID NO: 7, or a sequence having one or more conservative amino acid substitutions as compared to the sequence set forth in SEQ ID NO: 7; and/or
a light chain variable region of the antibody comprises a sequence set forth in SEQ ID NO: 8, or a sequence having at least 80% identity to the sequence set forth in SEQ ID NO: 8, or a sequence having one or more conservative amino acid substitutions as compared to the sequence set forth in SEQ ID NO: 8.

15. The antibody according to claim 14, wherein a heavy chain of the antibody comprises a sequence set forth in SEQ ID NO: 9, or a sequence having at least 80% identity to the sequence set forth in SEQ ID NO: 9, or a sequence having one or more conservative amino acid substitutions as compared to the sequence set forth in SEQ ID NO: 9; and/or
a light chain of the antibody comprises a sequence set forth in SEQ ID NO: 10, or a sequence having at least 80% identity to the sequence set forth in SEQ ID NO: 10, or a sequence having one or more conservative amino acid substitutions as compared to the sequence set forth in SEQ ID NO: 10.

16. The antibody composition according to any one of claims 11-15, wherein the antibody is expressed by a CHO cell with α-(1,6)-fucosyltransferase gene FUT8 knocked out.

17. A pharmaceutical composition, comprising the antibody according to any one of claims 1-10 or the antibody composition according to any one of claims 11-16 and a pharmaceutically acceptable carrier.

18. A method for treating a cell proliferative disease, comprising administering to a patient an effective dose of the anti-HER2 antibody according to any one of claims 1-10, or the antibody composition according to any one of claims 11-15, or the pharmaceutical composition according to claim 17.

19. The method according to claim 18, wherein the effective dose of the anti-HER2 antibody or the antibody composition is from 50 mg to 1000 mg per dose.

20. The method according to claim 18 or 19, wherein the patient is administered 1-15 mg/kg of the anti-HER2 antibody or the antibody composition per treatment cycle.

21. The method according to any one of claims 18-20, wherein the treatment cycle of the anti-HER2 antibody or the antibody composition is administered once every 1 week to 5 weeks.

22. The method according to any one of claims 18-21, further comprising administering to a patient an effective dose of a therapeutic agent selected from the group consisting of: trastuzumab, trastuzumab-hyaluronidase, docetaxel, a chemotherapeutic agent and a cytokine.

23. The method according to any one of claims 18-22, wherein the cell proliferative disease is cancer.
